# EUROPEAN PATENT APPLICATION

(11) **EP 4 382 531 A1**
(43) Date of publication of application: **12.06.2024**
(21) Application number: 22852224.9
(22) Date of filing: 03.08.2022
(51) Int. Cl.: C07J 71/00, A61K 31/7048, A61P 29/00, A61P 11/00, A61P 11/06, A61P 11/08

(54) **CRYSTAL FORMS OF KUDING SAPONIN A COMPOUND, PHARMACEUTICAL COMPOSITION AND USE THEREOF**

(30) Priority: 04.08.2021 CN 202110891737
(71) Applicant: Shanghai KE Pharmaceutical Co., Ltd, Shanghai 200023 (CN)
(72) Inventor: MA, Ming, Shanghai 200023 (CN)
(74) Representative: Pace Napoleone, Maria
(86) International application number: PCT/CN2022/109901
(87) International publication number: WO 2023/011512

(57) **Abstract**

The present invention provides crystal forms of the compound Kuding saponin A, and a pharmaceutical composition and a use thereof. The crystal forms comprise crystal forms A, B, C, D, E, G, J, K and L. Characteristic peaks in the X-ray powder diffraction (XRPD) pattern of each crystal form are as described in the application. The crystal forms of the present invention are stable under suitable conditions, especially crystal forms A, C and J. The crystal forms of the present invention can be used to prepare a pharmaceutical composition for treating pulmonary disease.

## Description

### TECHNICAL FIELD

The present invention relates to the technical fields of chemical medicine and crystal preparation, and in particular to crystal forms of a Kudinoside A compound and a pharmaceutical composition and use thereof.

### BACKGROUND

*Ilex kudingcha* C. J. Tseng is the tender leaf or leaf of *Ilex kudingcha* C. J. Tseng, a plant in the genus *Ilex* of *Aquifoliaceae* family, and it is a folk traditional medicinal plant in south and southwest China and has wide application and a long history. It has the effects of clearing away heat and toxic materials, sterilizing and diminishing inflammation, invigorating stomach and resolving food stagnation, relieving cough and reducing sputum, helping produce saliva and slake thirst, refreshing the mind, and improving eyesight and intelligence, and can be used for treating headache, toothache, conjunctival congestion, polydipsia due to heat disease, dysentery, etc.; it also has the effects of promoting blood circulation and regulating blood lipid and can be used for treating hypertension, hyperlipemia, etc.; it also has the effect of dilating bronchial smooth muscle and is suitable for treating various respiratory tract and pulmonary diseases (such as COPD and asthma).

Various Kudinoside compounds (such as Kudinoside A) with the effects described above can be extracted and separated from *Ilex kudingcha* C. J. Tseng, which have been described in a large number of documents. For example, reference may be made to Tu Pengfei et al., "Research on chemical components of *Ilex kudingcha* C. J. Tseng and its effect in reducing blood lipids", The 25th Chinese Chemical Society Congress, 2006. The structure of the Kudinoside A, namely 3*β*-12*α*-19*α*-trihydroxy-ursane-13(18)-ene-28,20*β*-lactone-3-O-[*β*-D-glucosyl-(1→3)-[*α*-L-rham nosyl-(1→2)]-*α*-L-arabinoside, is shown as formula (I):

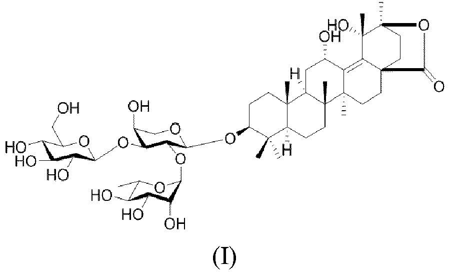

Different crystal forms of a medicinal compound may result in different physical and chemical properties, as well as differences in the solubility and stability, so that the degradation and absorption of the medicinal compound *in vivo* are affected, and thereby the clinical efficacy of the medicinal compound is different. Therefore, in the development of medicinal compounds, there is a need to comprehensively and systematically screen different crystal forms and explore their *in vivo* utility to select the crystal form that is most suitable for development.

### SUMMARY

The present invention obtains 11 crystal forms of Kudinoside A by screening in various solvent systems by using various crystallization modes, and the crystal forms are marked as crystal forms A, B, C, D, E, G, I₁, I₂, J, K, and L. Among them, the crystal form I₁ and the crystal form I₂ are of poor crystallinity (or called low crystallinity) and cannot exist stably.

Accordingly, the present invention provides crystal forms A, B, C, D, E, G, J, K, and L of Kudinoside A. The present invention also comprises mixtures of any two or more of the described crystal forms in any ratio. The crystal forms of the present invention can exist stably under suitable conditions, particularly the crystal forms A, C, and J, which can exist stably as a dry powder at room temperature. Thus, in a preferred embodiment, the present invention provides crystal forms A, C, and J of Kudinoside A and any mixture thereof.

In particular, the present invention provides a crystal form A of a Kudinoside A compound, wherein the crystal form A has an X-ray powder diffraction (XRPD) pattern comprising characteristic peaks at 2θ angles of 6.7°±0.2°, 7.3°±0.2°, 7.9°±0.2°, 10.5°±0.2°, 10.9°±0.2°, and 14.1°±0.2°.

In a preferred embodiment, the X-ray powder diffraction (XRPD) pattern of the crystal form A further comprises characteristic peaks at 2θ angles of 3.6°±0.2°, 12.8°±0.2°, 13.2°±0.2°, 13.6°±0.2°, 15.2°±0.2°, 16.1°±0.2°, 18.3°±0.2°, and 24.1°±0.2°.

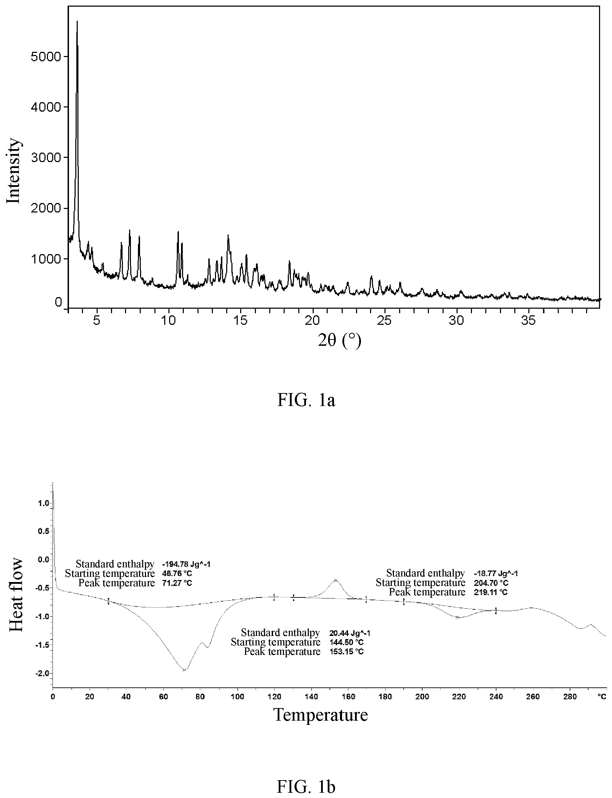

In a preferred embodiment, the crystal form A has an X-ray powder diffraction (XRPD) pattern substantially as shown in FIG. 1a.

In a preferred embodiment, the X-ray powder diffraction (XRPD) pattern of the crystal form A comprises characteristic peaks at 2θ angles shown in Table 1:

**Table 1. List of X-ray powder diffraction (XRPD) peaks of crystal form A**

| **2Θ°** | **d-spacing** | **Relative intensity (%)** |
|---|---|---|
| 3.617 | 24.4057 | 100 |
| 4.396 | 20.0831 | 8.8 |
| 5.417 | 16.302 | 4.3 |
| 6.309 | 13.998 | 2.2 |
| 6.702 | 13.1788 | 15.2 |
| 7.28 | 12.1329 | 22.2 |
| 7.919 | 11.1546 | 19.8 |
| 8.843 | 9.9916 | 3.3 |
| 10.639 | 8.3085 | 24.8 |
| 10.899 | 8.1112 | 19.8 |
| 11.298 | 7.8252 | 5.5 |
| 12.523 | 7.0627 | 2.7 |
| 12.765 | 6.929 | 11.2 |
| 13.322 | 6.6405 | 9.6 |
| 13.642 | 6.4858 | 11.8 |
| 14.105 | 6.2737 | 20.9 |
| 14.708 | 6.0177 | 3.1 |
| 15.045 | 5.884 | 9.3 |
| 15.383 | 5.7553 | 13.7 |
| 16.105 | 5.4988 | 10.4 |
| 16.589 | 5.3394 | 6.2 |
| 17.168 | 5.1608 | 3.3 |
| 17.662 | 5.0175 | 4.3 |
| 18.349 | 4.831 | 12.1 |
| 18.689 | 4.7441 | 7.8 |
| 19.005 | 4.6657 | 7.2 |
| 19.285 | 4.5987 | 4.4 |
| 19.664 | 4.5109 | 7.4 |
| 20.548 | 4.3188 | 3.6 |
| 20.814 | 4.2642 | 3.5 |
| 21.408 | 4.1472 | 3.4 |
| 22.387 | 3.968 | 5.3 |
| 23.025 | 3.8594 | 1.9 |
| 23.366 | 3.8039 | 1.4 |
| 23.559 | 3.7732 | 2.3 |
| 24.067 | 3.6946 | 7.9 |
| 24.625 | 3.6122 | 5.7 |
| 25.148 | 3.5382 | 3.3 |
| 25.351 | 3.5104 | 4.1 |
| 26.065 | 3.4158 | 5.4 |
| 27.57 | 3.2327 | 3.4 |
| 28.573 | 3.1214 | 3.1 |
| 28.994 | 3.0771 | 2 |
| 30.269 | 2.9503 | 3.1 |
| 31.535 | 2.8347 | 1.1 |
| 32.408 | 2.7603 | 1.3 |
| 33.276 | 2.6903 | 2.3 |
| 33.618 | 2.6637 | 2.6 |
| 34.875 | 2.5704 | 2 |
| 37.24 | 2.4125 | 1.2 |
| 37.677 | 2.3855 | 1.4 |
| 38.671 | 2.3264 | 1.1 |
| 39.072 | 2.3035 | 1.2 |

In a preferred embodiment, the crystal form A has a differential scanning calorimetry (DSC) graph substantially as shown in FIG. 1b.

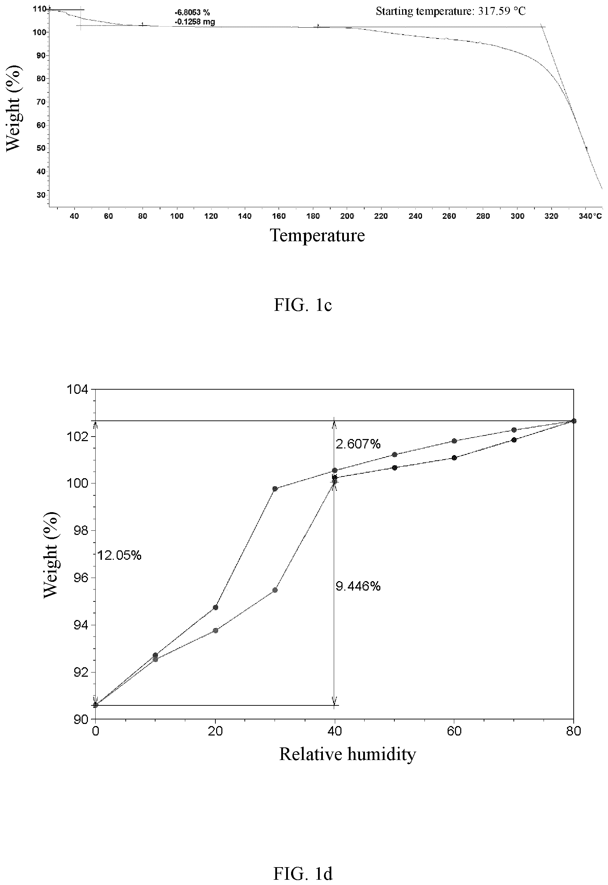

In a preferred embodiment, the crystal form A has a thermogravimetric analysis (TGA) graph substantially as shown in FIG. 1c.

In a preferred embodiment, the crystal form A has a dynamic vapor sorption (DVS) graph substantially as shown in FIG. 1d.

The crystal form C of the Kudinoside A compound provided herein has an X-ray powder diffraction (XRPD) pattern comprising characteristic peaks at 2θ angles of 7.8°±0.2°, 12.7°±0.2°, 14.4°±0.2°, 14.9°±0.2°, 16.3°±0.2°, 18.6°±0.2°, and 25.1°±0.2°.

In a preferred embodiment, the X-ray powder diffraction (XRPD) pattern of the crystal form C further comprises characteristic peaks at 2θ angles of 3.9°±0.2°, 8.3°±0.2°, 13.2°±0.2°, 19.5°±0.2°, and 20.3°±0.2°.

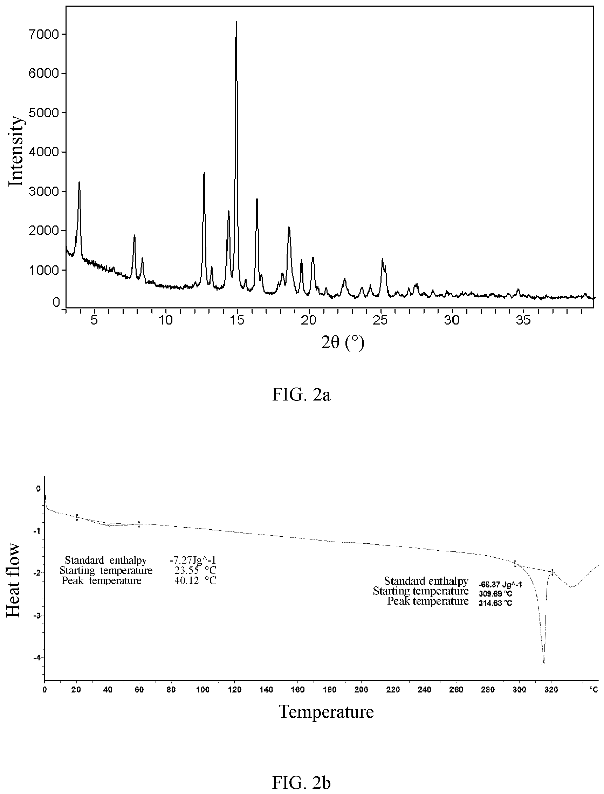

In a preferred embodiment, the crystal form C has an X-ray powder diffraction (XRPD) pattern substantially as shown in FIG. 2a.

In a preferred embodiment, the X-ray powder diffraction (XRPD) pattern of the crystal form C comprises characteristic peaks at 2θ angles shown in Table 2:

**Table 2. List of X-ray powder diffraction (XRPD) peaks of crystal form C**

| **2Θ°** | **d-spacing** | **Relative intensity (%)** |
|---|---|---|
| 3.921 | 22.5186 | 28.5 |
| 6.329 | 13.9538 | 2.1 |
| 7.802 | 11.3216 | 16.4 |
| 8.343 | 10.5886 | 8.1 |
| 12.042 | 7.3433 | 1.5 |
| 12.664 | 6.9841 | 43.1 |
| 13 203 | 6.7 | 7.9 |
| 14.364 | 6.1611 | 28.8 |
| 14.886 | 5.9461 | 100 |
| 15.548 | 5.6944 | 3.4 |
| 16.346 | 5.4184 | 34.8 |
| 16.647 | 5.3209 | 6.6 |
| 18.109 | 4.8946 | 7.4 |
| 18.587 | 4.7698 | 24.2 |
| 19.466 | 4.5564 | 12.4 |
| 20.266 | 4.3782 | 13.6 |
| 20.587 | 4.3107 | 3.3 |
| 21.185 | 4.1903 | 2.8 |
| 21.981 | 4.0404 | 1.1 |
| 22.449 | 3.9572 | 7.1 |
| 23.725 | 3.7471 | 3.6 |
| 24.285 | 3.662 | 4.3 |
| 25.09 | 3.5463 | 13.9 |
| 26.171 | 3.4023 | 2.1 |
| 26.456 | 3.3662 | 0.9 |
| 26.949 | 3.3057 | 2.6 |
| 27.509 | 3.2397 | 4.7 |
| 28.01 | 3.1829 | 1.2 |
| 28.651 | 3.1131 | 2.5 |
| 29.593 | 3.0161 | 2.4 |
| 29.909 | 2.985 | 1.6 |
| 30.247 | 2.9524 | 0.9 |
| 30.668 | 2.9128 | 1.9 |
| 30.91 | 2.8905 | 1.6 |
| 31.433 | 2.8437 | 1.4 |
| 32.366 | 2.7638 | 1 |
| 32.869 | 2.7226 | 1.5 |
| 33.933 | 2.6396 | 1.6 |
| 34.595 | 2.5907 | 32 |
| 34.957 | 2.5646 | 1.2 |
| 35.325 | 2.5388 | 0.8 |
| 35.874 | 2.5011 | 1 |
| 39.254 | 2.2932 | 2 |

In a preferred embodiment, the crystal form C has a differential scanning calorimetry (DSC) graph substantially as shown in FIG. 2b.

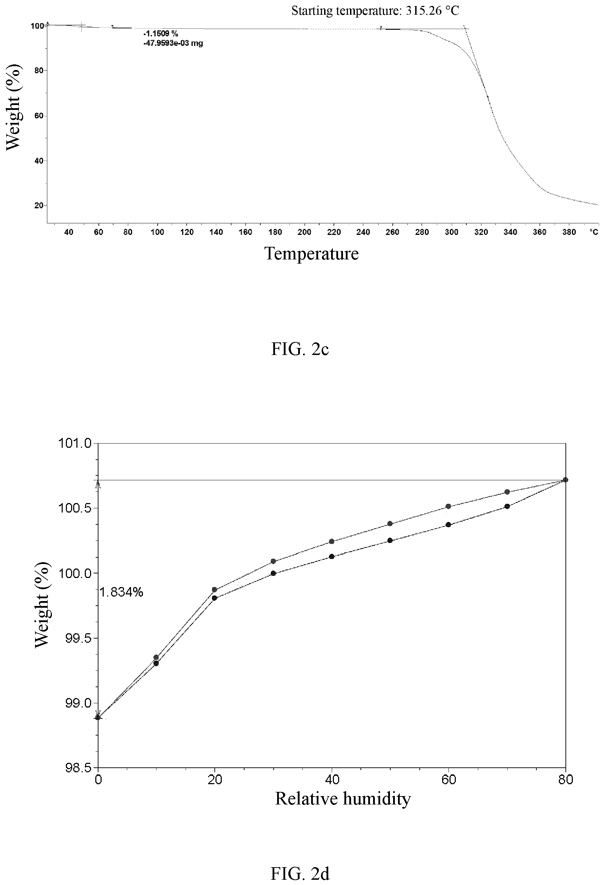

In a preferred embodiment, the crystal form C has a thermogravimetric analysis (TGA) graph substantially as shown in FIG. 2c.

In a preferred embodiment, the crystal form C has a dynamic vapor sorption (DVS) graph substantially as shown in FIG. 2d.

The crystal form J of the Kudinoside A compound provided herein has an X-ray powder diffraction (XRPD) pattern comprising characteristic peaks at 2θ angles of 7.7°±0.2°, 9.3°±0.2°, 12.1°±0.2°, 14.1°±0.2°, 15.1°±0.2°, and 16.6°±0.2°.

In a preferred embodiment, the X-ray powder diffraction (XRPD) pattern of the crystal form J further comprises characteristic peaks at 2θ angles of 3.5°±0.2°, 4.7°±0.2°, 12.7°±0.2°, 13.7°±0.2°, 15.5°±0.2°, 16.2°±0.2°, and 18.1°±0.2°.

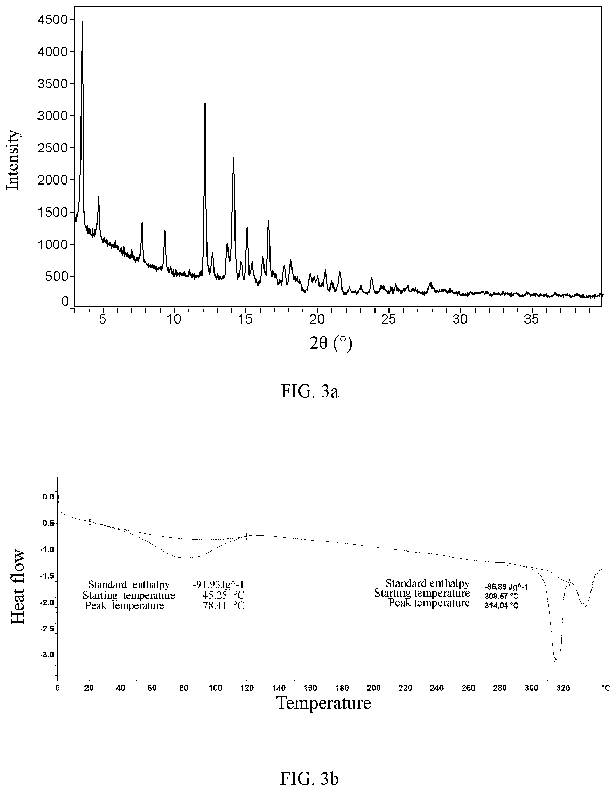

In a preferred embodiment, the crystal form J has an X-ray powder diffraction (XRPD) pattern substantially as shown in FIG. 3a.

In a preferred embodiment, the X-ray powder diffraction (XRPD) pattern of the crystal form J comprises characteristic peaks at 2θ angles shown in Table 3:

**Table 3. List of X-ray powder diffraction (XRPD) peaks of crystal form J**

| **2Θ°** | **d-spacing** | **Relative intensity (%)** |
|---|---|---|
| 3.542 | 24.9274 | 100 |
| 4.663 | 18.9364 | 19.7 |
| 5.859 | 15.0718 | 3.6 |
| 6.461 | 13.668 | 3.3 |
| 7.001 | 12.6151 | 3.6 |
| 7.721 | 11.4402 | 19.9 |
| 9.323 | 9.4783 | 19.5 |
| 11.049 | 8.0008 | 2.8 |
| 12.143 | 7.2825 | 85.5 |
| 12.662 | 6.9853 | 12.2 |
| 13.703 | 6.4568 | 16.8 |
| 14.143 | 6.2571 | 59.1 |
| 14.663 | 6.0362 | 7.4 |
| 15.103 | 5.8614 | 25.3 |
| 15.46 | 5.7266 | 9.5 |
| 16.186 | 5.4716 | 13 |
| 16.565 | 5.3471 | 30.7 |
| 17.666 | 5.0164 | 7.9 |
| 18.106 | 4.8955 | 12.7 |
| 18.567 | 4.7748 | 5 |
| 18.767 | 4.7244 | 4.2 |
| 19.507 | 4.5469 | 7.6 |
| 19.782 | 4.4842 | 6.5 |
| 20.006 | 4.4346 | 7 |
| 20.526 | 4.3234 | 8.8 |
| 21.009 | 4.225 | 4.3 |
| 21.53 | 4.1239 | 9.8 |
| 22.264 | 3.9896 | 3.3 |
| 22.988 | 3.8656 | 3.7 |
| 23.734 | 3.7458 | 7.3 |
| 24.412 | 3.6433 | 3.4 |
| 25.116 | 3.5427 | 2.3 |
| 25.451 | 3.4968 | 4 |
| 26.292 | 3.3869 | 3.5 |
| 26.611 | 3.347 | 2.3 |
| 27.892 | 3.1961 | 4.7 |
| 29.254 | 3.0503 | 2 |
| 31.571 | 2.8315 | 1.8 |
| 31.876 | 2.8052 | 1.6 |
| 33.033 | 2.7095 | 1.8 |
| 33.876 | 2.6439 | 1.7 |
| 34.634 | 2.5878 | 2 |
| 35.966 | 2.4949 | 1.5 |
| 36.576 | 2.4548 | 1.7 |
| 38.087 | 2.3607 | 1.5 |

In a preferred embodiment, the crystal form J has a differential scanning calorimetry (DSC) graph substantially as shown in FIG. 3b.

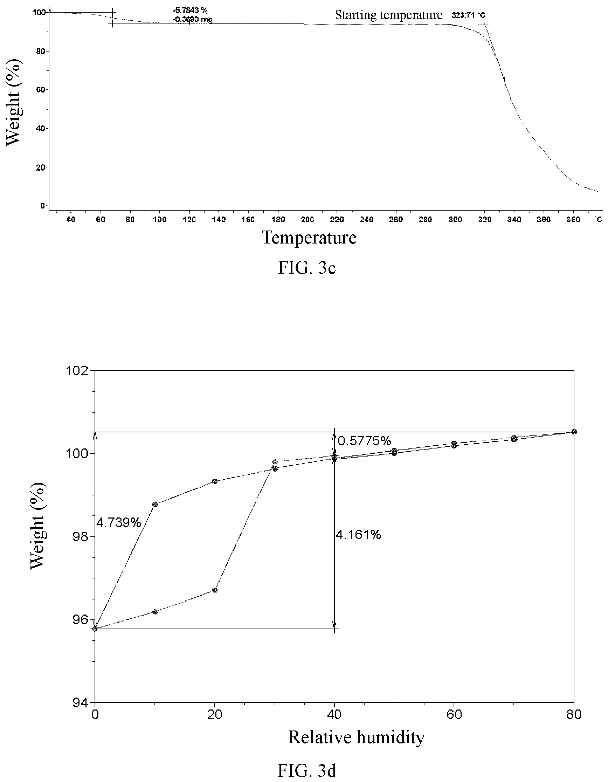

In a preferred embodiment, the crystal form J has a thermogravimetric analysis (TGA) graph substantially as shown in FIG. 3c.

In a preferred embodiment, the crystal form J has a dynamic vapor sorption (DVS) graph substantially as shown in FIG. 3d.

The crystal form B of the Kudinoside A provided herein has an X-ray powder diffraction (XRPD) pattern comprising characteristic peaks at 2θ angles of 7.4°±0.2°, 7.9°±0.2°, 11.8°±0.2°, 12.5°±0.2°, 14.3°±0.2°, and 15.4°±0.2°.

In a preferred embodiment, the X-ray powder diffraction (XRPD) pattern of the crystal form B further comprises characteristic peaks at 2θ angles of 4.8°±0.2°, 8.1°±0.2°, and 14.6°±0.2°.

In a preferred embodiment, the X-ray powder diffraction (XRPD) pattern of the crystal form B comprises characteristic peaks at 2θ angles shown in Table 14.

The crystal form D of the Kudinoside A provided herein has an X-ray powder diffraction (XRPD) pattern comprising characteristic peaks at 2θ angles of 6.2°±0.2°, 9.9°±0.2°, and 12.4°±0.2°.

In a preferred embodiment, the X-ray powder diffraction (XRPD) pattern of the crystal form D further comprises characteristic peaks at 2θ angles of 4.5°±0.2°, 7.2°±0.2°, 12.9°±0.2°, and 19.0°±0.2°.

In a preferred embodiment, the X-ray powder diffraction (XRPD) pattern of the crystal form D comprises characteristic peaks at 2θ angles shown in Table 15.

The crystal form E of the Kudinoside A provided herein has an X-ray powder diffraction (XRPD) pattern comprising characteristic peaks at 2θ angles of 5.6°±0.2°, 11.1°±0.2°, and 14.4°±0.2°.

In a preferred embodiment, the X-ray powder diffraction (XRPD) pattern of the crystal form E further comprises characteristic peaks at 2θ angles of 4.5°±0.2°, 7.7°±0.2°, 13.6°±0.2°, 18.1°±0.2°, 19.8°±0.2°, 20.8°±0.2°, and 28.0°±0.2°.

In a preferred embodiment, the X-ray powder diffraction (XRPD) pattern of the crystal form E comprises characteristic peaks at 2θ angles shown in Table 16.

The crystal form G of the Kudinoside A provided herein has an X-ray powder diffraction (XRPD) pattern comprising characteristic peaks at 2θ angles of 5.7°±0.2°, 7.8°±0.2°, 9.2°±0.2°, 9.9°±0.2°, 17.5°±0.2°, 19.8°±0.2°, 20.0°±0.2°, 28.5°±0.2°, and 37.8°±0.2°.

In a preferred embodiment, the X-ray powder diffraction (XRPD) pattern of the crystal form G further comprises characteristic peaks at 2θ angles of 3.4°±0.2°, 7.3°±0.2°, 12.0°±0.2°, 14.4°±0.2°, 17.0°±0.2°, 18.8°±0.2°, 22.5°±0.2°, 25.5°±0.2°, 25.7°±0.2°, and 26.1°±0.2°.

In a preferred embodiment, the X-ray powder diffraction (XRPD) pattern of the crystal form G comprises characteristic peaks at 2θ angles shown in Table 17.

The crystal form K of the Kudinoside A provided herein has an X-ray powder diffraction (XRPD) pattern comprising characteristic peaks at 2θ angles of 7.6°±0.2°, 11.1°±0.2°, 12.1°±0.2°, and 14.3°±0.2°.

In a preferred embodiment, the X-ray powder diffraction (XRPD) pattern of the crystal form K further comprises characteristic peaks at 2θ angles of 4.5°±0.2°, 4.9°±0.2°, 5.5°±0.2°, 8.2°±0.2°, 13.5°±0.2°, and 18.7°±0.2°.

In a preferred embodiment, the X-ray powder diffraction (XRPD) pattern of the crystal form K comprises characteristic peaks at 2θ angles shown in Table 20.

The crystal form L of the Kudinoside A provided herein has an X-ray powder diffraction (XRPD) pattern comprising characteristic peaks at 2θ angles of 9.5°±0.2°, 12.3°±0.2°, 13.6°±0.2°, 14.5°±0.2°, 15.4°±0.2°, and 16.2°±0.2°.

In a preferred embodiment, the X-ray powder diffraction (XRPD) pattern of the crystal form L further comprises characteristic peaks at 2θ angles of 3.7°±0.2°, 7.9°±0.2°, 16.9°±0.2°, 17.3°±0.2°, 18.8°±0.2°, and 20.4°±0.2°.

In a preferred embodiment, the X-ray powder diffraction (XRPD) pattern of the crystal form L comprises characteristic peaks at 2θ angles shown in Table 21.

The present invention also provides methods for preparing the crystal forms A, C, and J of the Kudinoside A compound.

In a preferred embodiment, the crystal form A of the Kudinoside A can be obtained by leaching and purifying *kudingcha* powder and then crystallizing in a specific solvent.

Preferably, the method for preparing the crystal form A is as follows: subjecting *kudingcha* powder to percolation extraction and concentrating to obtain an extract in the form of an extractum; diluting and centrifuging the extractum to obtain a crude sample; purifying the crude sample twice by high-pressure chromatography, decoloring with active carbon, recrystallizing, freeze-drying, and finally crushing to obtain a crystal form A of Kudinoside A (i.e., crystal form A).

In a preferred embodiment, the percolation extraction is carried out using an alcohol solvent (e.g., ethanol). For example, the percolation extraction is carried out using pure ethanol or 50-90% (v/v) aqueous ethanol solution. Generally, the mass ratio of the *kudingcha* powder to the alcohol is 1:2.5-1: 1.5. The extract in the form of an extractum can be diluted with water and anhydrous ethanol successively and centrifuged to obtain a crude sample. The amount of water can be 0.5-1 times the mass of the *kudingcha* powder, and the amount of anhydrous ethanol can be 0.3-0.8 times the mass of the *kudingcha* powder.

Preferably, the crude sample is dissolved in alcohol and recrystallized at a temperature not more than 10 °C. The alcohol may be ethanol or an aqueous solution thereof, such as a 20-50% (v/v) ethanol solution. The dissolving is carried out at a relatively high temperature, e.g., 60-90 °C. The crystal obtained may be freeze-dried (e.g., at about 0 °C), then the temperature is raised to room temperature (e.g., about 20-28 °C) for drying, and finally the crystal is dried at a temperature, e.g., not less than 32 °C, thus obtaining the crystal form A. Generally, the freeze-drying may last for 1-3 h, the room temperature drying may last for about 3-5 h, and the drying at a temperature not less than 32 °C may last for 5-8 h.

In another preferred embodiment, the crystal form A of the Kudinoside A can be obtained by crystallizing amorphous Kudinoside A in a specific solvent.

Preferably, the method for preparing the crystal form A may be any one of the following three methods:
method 1: dissolving amorphous Kudinoside A in an alcohol solvent to obtain a dissolution solution of Kudinoside A; concentrating the dissolution solution until a white precipitate is separated out and no liquid drops are generated at an inlet of a distilled liquid collecting container, thus reaching the end point of concentration and obtaining a concentrated solution; and freeze-drying the concentrated solution to obtain the crystal form A;
method 2: dissolving amorphous Kudinoside A in an alcohol solvent to obtain a dissolution solution of Kudinoside A; subjecting the dissolution solution to sonication treatment and filtering to obtain a filtrate; and concentrating the filtrate under reduced pressure to obtain a dry solid, namely the crystal form A;
method 3: dissolving amorphous Kudinoside A in an alcohol solvent to obtain a dissolution solution of Kudinoside A, and naturally volatilizing the dissolution solution at room temperature to obtain a dry solid, namely the crystal form A.

Preferably, the alcohol solvent is methanol or ethanol. Preferably, in the methods 1 and 2, ethanol, such as an aqueous ethanol solution (e.g., a 70% ethanol solution), is used to dissolve the amorphous Kudinoside A. Preferably, in the method 3, methanol is used to dissolve the amorphous Kudinoside A. There is no particular limitation on the amount of the alcohol solvent used, and the amount should be sufficient to completely dissolve the amorphous Kudinoside A. Preferably, in the method 1, after the dissolution solution of the Kudinoside A is obtained, firstly filtering is carried out to obtain a clear solution, and then the clear solution is concentrated. A filter membrane with filtration pore of less than 5 µm is used for filtration. The dissolution solution or the clear solution thereof may be concentrated using a rotary evaporator. The concentrating may be carried out in a water bath at 50-60 °C, the rotating speed may be controlled to be 30-80 rpm, and the vacuum degree may be controlled to be not lower than 0.08 MPa.

In a preferred embodiment, the crystal form C of the Kudinoside A can be obtained by recrystallizing the crystal form A in a specific solvent.

Preferably, the method for preparing the crystal form C may be any one of the following three methods:
method 4: dissolving the crystal form A in a solvent (e.g., acetonitrile and isopropyl ether) to obtain a suspension; stirring for 3-5 days at room temperature and centrifuging; and drying the resulting solid under vacuum at 25-30 °C overnight to obtain the crystal form C;
method 5: dissolving the crystal form A in ethyl acetate to obtain a suspension; stirring for 2-4 days at room temperature and centrifuging; and drying the resulting solid under vacuum at 25-30 °C overnight to obtain the crystal form C;
method 6: heating the crystal form A for 3-10 min at 150-200 °C to obtain the crystal form C. Preferably, in the method 4, the volume ratio of acetonitrile to isopropyl ether is 1:1 to 1:5. Preferably, the mass-to-volume ratio (g:mL) of the crystal form A to the solvent is 1:20 to 1:50. Preferably, in the method 5, the mass-to-volume ratio (g:mL) of the crystal form A to ethyl acetate is 1:10 to 1:30.

Preferably, in the method 6, the crystal form A is heated at 170±5 °C and subjected to heat preservation for 3-7 min.

In a preferred embodiment, the crystal form J of the Kudinoside A can be obtained by recrystallizing the crystal form A in a specific solvent.

Preferably, the method for preparing the crystal form J may be any one of the following two methods:
method 7: dissolving the crystal form A in a saturated aqueous ethyl acetate solution to obtain a suspension; stirring for 3-5 days at room temperature and centrifuging; and drying the resulting solid under vacuum at 25-30 °C overnight to obtain the crystal form J;
method 8: dissolving the crystal form A in water to obtain a suspension; stirring for 2-4 days at room temperature and centrifuging; and drying the resulting solid under vacuum at 25-30 °C overnight to obtain the crystal form J.

Preferably, in the method 7, the mass-to-volume ratio (g:mL) of the crystal form A to the saturated aqueous ethyl acetate solution is 1:20 to 1:50.

Preferably, in the method 8, the mass-to-volume ratio (g:mL) of the crystal form A to water is 1:10 to 1:30.

In another preferred embodiment, the crystal form J of the Kudinoside A can be obtained by crystallizing amorphous Kudinoside A in a specific solvent.

Preferably, the method for preparing the crystal form J may be any one of the following two methods:
method 9: dissolving amorphous Kudinoside A in an alcohol solvent; adding water, stirring overnight at room temperature, and centrifuging; and drying the resulting solid under vacuum at 25-30 °C overnight to obtain the crystal form J;
method 10: dissolving amorphous Kudinoside A in an alcohol solvent and water; stirring overnight at 0-10 °C and centrifuging; and drying the resulting solid under vacuum at 25-30 °C overnight to obtain the crystal form J.

Preferably, in the method 9, the alcohol solvent is an anhydrous alcohol solvent, such as anhydrous ethanol. The mass-to-volume ratio (g:mL) of the Kudinoside A to the alcohol solvent is 1:30 to 1:100. The amount of water added may be 1-10 times the volume of the alcohol solvent. Typically, a white solid is separated out upon addition of water, at which time the mixture is stirred overnight at room temperature.

Preferably, in the method 10, the alcohol solvent is preferably an anhydrous alcohol solvent, such as anhydrous ethanol. The volume ratio of the alcohol solvent to water may be 1:1 to 1:5. The mass-to-volume ratio (g:mL) of the Kudinoside A to the alcohol solvent is 1:10 to 1:50. After the alcohol solvent, water and the Kudinoside A are mixed, the mixture may be heated in a water bath at, e.g., 60-80 °C, until the Kudinoside A is completely dissolved. Typically the mixture is stirred overnight at about 4 °C.

In some embodiments, the crystal form B is prepared from the crystal form A. Specifically, the crystal form A is heated to 110-130 °C and subjected to heat preservation for 3-10 min, thus obtaining the crystal form B.

In some embodiments, an alcohol solvent (e.g., isopropanol) and Kudinoside A are mixed, then the Kudinoside A is allowed to dissolve completely in a water bath at, e.g., not less than 55 °C and a solid is slowly separated out, and the mixture is slowly cooled to room temperature, stirred for 3-6 days, and then centrifuged, thus obtaining the crystal form D. The mass-to-volume ratio (mg:mL) of the alcohol solvent to the Kudinoside A may be in the range of 4-8: 1.

In some embodiments, Kudinoside A, an alcohol solvent, and water are mixed, then the Kudinoside A is dissolved at a temperature not less than 60 °C, and then the solution is slowly cooled to room temperature and left to stand to separate out a solid, thus obtaining the crystal form E. The alcohol solvent may be, e.g., anhydrous ethanol. There is no particular limitation on the amount of the alcohol solvent and water used, as long as the amount is sufficient to dissolve the Kudinoside A.

In some embodiments, Kudinoside A, an organic solvent (such as tetrahydrofuran), and water are mixed, then the Kudinoside A is dissolved completely, and then the solvent is allowed to evaporate at room temperature, thus obtaining the crystal form G. There is no particular limitation on the amount of the organic solvent and water used, as long as the amount is sufficient to dissolve the Kudinoside A. Of course, if the amount of the solvent is too large, the volatilization time is affected, resulting in too long preparation time. The specific amount can be determined by those skilled in the art according to actual production conditions.

In some embodiments, Kudinoside A is dissolved in anhydrous ethanol and then the solution is concentrated under reduced pressure and dried at 25-30 °C, thus obtaining the crystal form K.

In some embodiments, the crystal form J is heated to not less than 110 °C and subjected to heat preservation for 3-10 min, thus obtaining the crystal form L.

The present invention provides use of the crystal forms of the Kudinoside A compound in preparing a medicine for treating a pulmonary disease.

In a preferred embodiment, the pulmonary disease is chronic obstructive pulmonary disease or asthma.

In one embodiment, each crystal form of the Kudinoside A compound achieves therapeutic purposes by dilating trachea (bronchus); in another embodiment, each crystal form of the Kudinoside A compound achieves therapeutic purposes by resisting inflammation.

The present invention also provides a pharmaceutical composition for treating a pulmonary disease, wherein the pharmaceutical composition comprises one of or a mixture of more of the crystal forms A, B, C, D, E, G, J, K, and L of the Kudinoside A compound and a pharmaceutically acceptable carrier. Preferably, the pharmaceutical composition comprises any one or more of the crystal forms A, C, and J of the Kudinoside A. Preferably, in the pharmaceutical composition, the Kudinoside A compound is capable of maintaining the state of crystal form.

In a preferred embodiment, the crystal form of the Kudinoside A compound is the crystal form A, the crystal form C, or the crystal form J.

In a preferred embodiment, the pharmaceutical composition is in the dosage form of an inhalation formulation.

In a preferred embodiment, the inhalation formulation is an inhalation powder, an inhalation aerosol, an inhalation spray, an inhalation suspension, or an inhalation solution.

The term "pharmaceutically acceptable carrier" should be compatible with the Kudinoside A compound (crystal form) in the pharmaceutical composition of the present invention, that is, it is capable of being blended with the Kudinoside A compound without greatly reducing the effectiveness of the pharmaceutical composition in the treatment of a pulmonary disease (such as asthma and chronic obstructive pulmonary disease) in general.

In a preferred embodiment, the pharmaceutically acceptable carrier includes one or more of a lubricant, a glidant, a propellant, a solubilizer, a cosolvent, a diluent, a stabilizer, and a bacteriostatic agent.

In a preferred embodiment, the pharmaceutical carrier is lactose.

In another preferred embodiment, the pharmaceutical carrier is polyethylene glycol 200, ethanol, and 1,1,1,2-tetrafluoroethane (R134a).

In some embodiments, the present invention provides a method for dilating trachea (bronchus), which comprises administering to a subject in need thereof an effective amount of the crystal form of the Kudinoside A compound described in any of the embodiments herein, or a product prepared by the method described in any of the embodiments herein, or a pharmaceutical composition comprising the crystal form or the product. In some embodiments, the present invention provides a method for resisting inflammation, which comprises administering to a subject (particularly a subject having inflammation at the trachea, bronchus, and/or lung) in need thereof an effective amount of the crystal form of the Kudinoside A compound described in any of the embodiments herein, or a product prepared by the method described in any of the embodiments herein, or a pharmaceutical composition comprising the crystal form or the product. In some embodiments, the present invention provides a method for treating a pulmonary disease, which comprises administering to a subject in need thereof an effective amount of the crystal form of the Kudinoside A compound described in any of the embodiments herein, or a product prepared by the method described in any of the embodiments herein, or a pharmaceutical composition comprising the crystal form or the product. Preferably, in these methods, the crystal form is a crystal form A, a crystal form C, or a crystal form J, or any mixture thereof, and the product comprises the crystal form A, the crystal form C, or the crystal form J, or any mixture thereof. Preferably, in these methods, the subject is a subject having a pulmonary disease, more preferably a patient having chronic obstructive pulmonary disease or asthma.

### BRIEF DESCRIPTION OF THE DRAWINGS

FIG. 1a is an X-ray powder diffraction (XRPD) pattern of the crystal form A of Kudinoside A, wherein the abscissa is 2θ value (degree), and the ordinate is intensity (counts).
FIG. 1b is a differential scanning calorimetry (DSC) graph of the crystal form A of Kudinoside A, wherein the abscissa is temperature (°C), and the ordinate is heat flow (w/g).
FIG. 1c is a thermogravimetric analysis (TGA) graph of the crystal form A of Kudinoside A, wherein the abscissa is temperature (°C), and the ordinate is weight (%).
FIG. 1d is a dynamic vapor sorption (DVS) graph of the crystal form A of Kudinoside A, wherein the abscissa is relative humidity (%), and the ordinate is weight (%).
FIG. 2a is an X-ray powder diffraction (XRPD) pattern of the crystal form C of Kudinoside A, wherein the abscissa is 2θ value (degree), and the ordinate is intensity (counts).
FIG. 2b is a differential scanning calorimetry (DSC) graph of the crystal form C of Kudinoside A, wherein the abscissa is temperature (°C), and the ordinate is heat flow (w/g).
FIG. 2c is a thermogravimetric analysis (TGA) graph of the crystal form C of Kudinoside A, wherein the abscissa is temperature (°C), and the ordinate is weight (%).
FIG. 2d is a dynamic vapor sorption (DVS) graph of the crystal form C of Kudinoside A, wherein the abscissa is relative humidity (%), and the ordinate is weight (%).
FIG. 3a is an X-ray powder diffraction (XRPD) pattern of the crystal form J of Kudinoside A, wherein the abscissa is 2θ value (degree), and the ordinate is intensity (counts).
FIG. 3b is a differential scanning calorimetry (DSC) graph of the crystal form J of Kudinoside A, wherein the abscissa is temperature (°C), and the ordinate is heat flow (w/g).
FIG. 3c is a thermogravimetric analysis (TGA) graph of the crystal form J of Kudinoside A, wherein the abscissa is temperature (°C), and the ordinate is weight (%).
FIG. 3d is a dynamic vapor sorption (DVS) graph of the crystal form J of Kudinoside A, wherein the abscissa is relative humidity (%), and the ordinate is weight (%).
FIG. 4a: is an X-ray powder diffraction (XRPD) pattern of the crystal form J of Kudinoside A under conditions of the long-term test and the accelerated test, wherein the abscissa is 2θ value (degree), and the ordinate is intensity (counts).
FIG. 4b is a thermogravimetric analysis (TGA) graph of the crystal form J of Kudinoside A under conditions of the long-term test and the accelerated test, wherein the abscissa is temperature (°C), and the ordinate is weight (%).
FIG. 5a is an X-ray powder diffraction (XRPD) pattern of single crystal form A and crystal form J and an initial mixed sample of the two in a competition experiment of crystal form A and crystal form J. The abscissa is 2θ value (degree), and the ordinate is intensity (counts).
FIG. 5b is an X-ray powder diffraction (XRPD) pattern obtained after 3 days of competition of crystal form A and crystal form J. The abscissa is 2θ value (degree), and the ordinate is intensity (counts).

### DETAILED DESCRIPTION

In the following description, certain specific details are set forth to provide a thorough understanding of various embodiments of the present invention. However, it will be understood by those skilled in the art that the present invention may be put into practice without these details. The following description of several embodiments is provided with the understanding that the present disclosure is to be considered an exemplification of the claimed subject matter and is not intended to limit the appended claims to the specific embodiments illustrated. Headings used throughout this disclosure are provided for convenience only and should not be construed as limiting the claims in any way. Embodiments shown under any heading may be combined with embodiments shown under any other heading.

Throughout the specification and claims, unless otherwise required in the context, the word "comprise" and variants thereof are to be construed in an open, inclusive sense, i.e., "including but not limited to"; also, "comprise" and variants thereof such as "comprises" include "consisting essentially of ..." as well as "consisting of...".

Reference throughout this specification to "one embodiment" or "an embodiment" means that particular features, structures, or characteristics described based on the embodiment are included in at least one embodiment of the present invention. Thus, the phrase "in one embodiment" or "in an embodiment", when appearing in various places throughout this specification, does not necessarily refer to the same embodiment. Furthermore, the particular features, structures, or characteristics may be combined in any suitable manner in one or more embodiments.

Herein, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by FDA, NMPA, or other relevant agencies as being acceptable for use in humans or domestic animals.

Herein, "pharmaceutical composition" refers to a formulation of a compound of the present invention and a medium generally accepted in the art for the delivery of a biologically active compound to mammals, e.g., humans. Such a medium includes all pharmaceutically acceptable excipients therefor.

Herein, "effective amount" or "therapeutically effective amount" refers to an amount of a compound according to the present invention that, when administered to a patient in need thereof, is sufficient to effect treatment for disease states, conditions, or disorders for which the compounds have utility. Such an amount would be sufficient to elicit the biological or medical response of a tissue system or patient that is sought by a researcher or clinician. The amount of a compound according to the present invention that constitutes a therapeutically effective amount will vary depending on such factors as the compound and its biological activity, the composition used for administration, the time of administration, the route of administration, the rate of excretion of the compound, the duration of the treatment, the type of disease state or disorder being treated and its severity, drugs used in combination with or concurrently with the compounds of the present invention, and the age, body weight, general health, sex, and diet of the patient. Such a therapeutically effective amount can be determined routinely by one of ordinary skill in the art based on their own knowledge, the prior art, and the present disclosure. The term "treatment" or "treating", as used herein, unless otherwise indicated, means reversing, alleviating, or inhibiting the progression of the disorder or condition to which such term applies, or preventing such disorder or condition or one or more symptoms of such disorder or condition. "Prevention" or "preventing" means any treatment of a disease or condition that causes the clinical symptoms of the disease or condition not to develop.

Herein, "subject" or "patient" refers to an animal, such as a mammal (including a human), that has been or will be the object of treatment, observation, or experiment. The methods described herein may be useful in human therapy and/or veterinary applications. In some embodiments, the subject is a mammal (or the patient). In some embodiments, the subject (or the patient) is a human, a domestic animal (e.g., dogs and cats), a farm animal (e.g., cattle, horses, sheep, goats, and pigs), and/or a laboratory animal (e.g., mice, rats, hamsters, guinea pigs, pigs, rabbits, dogs, and monkeys). In some embodiments, the subject (or the patient) is a human. "Human (or patient) in need thereof refers to a human who may have or is suspect to have diseases or conditions that would benefit from certain treatment, for example, being treated with the compounds disclosed herein according to the present application.

Reference to "about" a value or parameter herein includes (and describes) embodiments that are directed to that value or parameter per se. For example, description referring to "about X" includes description of "X". Also, the singular forms "a" and "the" include plural references, unless otherwise clearly specified in the context. Thus, e.g., reference to "the compound" includes a plurality of such compounds and reference to "the assay" includes reference to one or more assays and equivalents thereof known to those skilled in the art.

Herein, "pharmaceutically acceptable" or "physiologically acceptable" refers to compounds, salts, compositions, dosage forms, and other materials that are useful in preparing a pharmaceutical composition that is suitable for veterinary or human pharmaceutical use.

Herein, "unit dosage form" is a physically discrete unit suitable as a unitary dosage for subjects (e.g., human subjects and other mammals), and each unit contains a predetermined quantity of active material calculated to produce the desired therapeutic effect, in association with a suitable pharmaceutical excipient.

Herein, "substantially as shown in", when referring to, e.g., an XRPD pattern, a DSC thermogram, a DVS graph, or a TGA graph, includes a pattern, a thermogram, or a graph that is not necessarily identical to those depicted herein but falls within the limits of experimental error or deviations when considered by one of ordinary skill in the art.

In some embodiments, with respect to a particular crystalline form of a compound, the term "substantially pure" or "substantially free" means that the composition comprising the crystalline form contains less than 20%, less than 15%, less than 10%, less than 5%, or less than 1% by weight of other substances, including other crystalline forms and/or impurities. In some embodiments, "substantially pure" or "substantially free" refers to a substance that is free of other materials, including other crystalline forms and/or impurities. Impurities may, e.g., include by-products or left-over reagents from chemical reactions, contaminants, degradation products, other crystalline forms, water, and solvents.

Herein, for the characteristic powder X-ray diffraction peak position of the crystal form, the allowable error of the angular position (2θ) is ±0.2°. This error is used when comparing two powder X-ray diffraction patterns. If a diffraction peak in one pattern is designated as a certain angular position range of determined peak position ±0.2° (2θ), and a diffraction peak in another pattern is designated as the other angular position range of determined peak position ±0.2° (2θ), and if these peak ranges overlap, the two peaks are considered to have the same angular position (2θ). For example, if a diffraction peak in a pattern is determined to be at 5.20°, for comparison, the allowable error allows the peak to be specified in the range of 5.00°-5.40°. If the control peak in another diffraction pattern is determined to be at 5.35°, for comparison, the allowable error allows the peak to be specified in the range of 5.15°-5.55°. Because of the overlap between the two peak position ranges, the two compared peaks are considered to have the same angular position (2θ). In some embodiments, the allowable error of the angular position (2θ) is ±0.1°.

Herein, "sonication" or "sonication treatment" is used to well mix or completely dissolve substances within the solution. The process parameters of sonication treatment are different according to different treatment objects and can be set by those skilled in the art according to actual use conditions. For example, the power of the sonication treatment may be set to 60-2000 W, the frequency may be set to 20-80 kHz, and the time may be set to 5-150 minutes.

In the present invention, a plurality of instrumental analysis methods are adopted. In one aspect, the crystal forms A, C, and J of the prepared Kudinoside A are identified (such as X-ray powder diffraction analysis); in another aspect, the properties of the crystal forms are explored (such as differential scanning calorimetry analysis, thermogravimetric analysis, and dynamic vapor sorption analysis).

X-ray powder diffraction (XRPD) is commonly used for crystal structure analysis, and its diffraction intensity is determined by the element species, number, and arrangement of atoms in the unit cells of crystals. Methods for determining X-ray powder diffraction of crystals are well known to those skilled in the art, and the present invention uses an X-ray powder diffractometer to determine the crystal forms A, C, and J of Kudinoside A, and the specific measurement conditions are shown in Table 4 (unless otherwise specified, the sample is not ground prior to detection):

**Table 4. X-ray powder diffractometer and measurement parameters thereof**

| **X-Ray Powder Diffractometer (XRPD)** | | |
|---|---|---|
| Instrument | Model | Bruker D8 Advance Diffractometer |
| | Technical indexes | Kα radiation (40 kV, 40 mA) with a copper target wavelength of 1.54 Å, θ-2θ goniometer, nickel plate filtration, Lynxeye detector |
| | Acquisition software | Diffrac Plus XRD Commander |
| | Calibration material | Corundum (Al₂O₃) |
| | Analysis software | MDI Jade |
| Parameter | Detection angle | 3-40° 2θ/3-30° 2θ (Heat stage XRPD) |
| | Step length | 0.02° 2θ |
| | Speed | 0.2s. step⁻¹ |
| | Amount of the sample to be detected | >2 mg |

Through determination, the crystal forms A, C, and J have specific characteristic peaks on an X-ray powder diffraction pattern; specifically, the crystal forms A, C, and J have characteristic peaks at 2θ angles shown in Tables 1, 2, and 3, respectively.

Differential scanning calorimetry (DSC) is an important thermal analysis method that uses the endothermic rate or the exothermic rate of a sample (mJ/s) as the ordinate and the temperature or time as the abscissa to determine various thermodynamic and kinetic parameters, such as crystallization rate, melting temperature, phase transition temperature of a substance, etc. Methods for performing differential scanning calorimetry analysis on the crystals are well known to those skilled in the art. In the present invention, the crystal forms A, C, and J of Kudinoside A are determined by using a differential scanning calorimeter, and the specific measurement conditions are shown in Table 5.

**Table 5. Differential scanning calorimeter and measurement parameters thereof**

| **Differential scanning calorimeter (DSC)** | |
|---|---|
| Model | METTLER TOLEDO DSC 3 |
| Controlling software | STARe |
| Analysis software | STARe |
| Sample tray | Aluminum crucible (with cover punched) |
| Amount of the sample to be detected | 1 mg-10 mg |
| Protective gas | Nitrogen |
| Gas flow rate | 50 mL/min |
| Common detection parameters | The initial set temperature being 0 °C; heating to 300 °C/350 °C at a rate of 10 °C/min |

The inventors mainly determine parameters such as the desolvation peak and melting point of the crystal forms A, C, and J of the Kudinoside A by using the method described above.

The differential scanning calorimetry analysis of the crystal form A is shown in FIG. 1b: the temperature range of its desolvation peak is about 30-120 °C, and its melting point is about 205 °C.

The differential scanning calorimetry analysis of the crystal form C is shown in FIG. 2b: the temperature range of its desolvation peak is about 20-60 °C, and its melting point is about 310 °C.

The differential scanning calorimetry analysis of the crystal form J is shown in FIG. 3b: the temperature range of its desolvation peak is about 20-120 °C, and its melting point is about 309 °C.

Thermogravimetric analysis, or TGA for short, refers to a thermal analysis method for measuring the relationship of the mass of a sample to be detected with the change of temperature or time at a programmed temperature, and it is used to study information related to the quality of the sample, such as thermal stability, components of intermediate products possibly generated, etc. It is often used in combination with other thermal analysis methods for comprehensive thermal analysis. Methods for performing thermogravimetric analysis on the crystals are well known to those skilled in the art. In the present invention, the crystal forms A, C, and J of Kudinoside A are determined by using a thermogravimetric analyzer, and the specific measurement conditions are shown in Table 6.

**Table 6. Thermogravimetric analyzer and measurement parameters thereof**

| **Thermogravimetric analyzer (TGA)** | |
|---|---|
| Model | METTLER TOLEDO TGA/DSC 3⁺ |
| Controlling software | STARe |
| Analysis software | STARe |
| Sample tray | Ceramic crucible |
| Amount of the sample to be detected | 1 mg-10 mg |
| Protective gas | Nitrogen |
| Gas flow rate | 50 mL/min |
| Common detection parameters | Stage I: the starting temperature being 25 °C, ramping at 10 K/min until reaching 150 °C. |
| | Stage 11: the starting temperature being 150 °C, ramping at 10 K/min until reaching 400 °C. |

The thermogravimetric analysis result of the crystal form A is shown in FIG. 1c: it loses about 6.8% of its weight before 80 °C and its decomposition temperature is about 318 °C.

The thermogravimetric analysis result of the crystal form C is shown in FIG. 2c: it loses about 1.2% of its weight before 70 °C and its decomposition temperature is about 315 °C.

The thermogravimetric analysis result of the crystal form J is shown in FIG. 3c: it loses about 5.8% of its weight before 120 °C and its decomposition temperature is about 324 °C.

Dynamic vapor sorption (DVS) is a test method for analyzing the moisture sorption performance of a sample and recording the moisture sorption isotherm, and it determines the stability of a compound by identifying its sorption capacity for moisture. Methods for performing dynamic vapor sorption analysis on the crystals are well known to those skilled in the art. In the present invention, the hygroscopicity of the crystal forms A, C, and J of Kudinoside A is determined by using a dynamic vapor sorption instrument, and the specific measurement conditions are shown in Table 7.

**Table 7. Dynamic vapor sorption instrument and measurement parameters thereof**

| **Dynamic vapor sorption (DVS) instrument** | | |
|---|---|---|
| Instrument | Model | TA Instruments Q5000TGA |
| | Controlling software | Thermal Advantage |
| | Analysis software | Universal Analysis |
| | Sample tray | Platinum crucible |
| Parameter | Amount of the sample to be detected | 1 mg-10 mg |
| | Protective gas | Nitrogen |
| | Gas flow rate | 10 mL/min |
| | Common detection parameters | Stage I: the starting temperature being 25 °C, the relative humidity being 0%, the isothermy being maintained for 300 min, and proceeding to the next stage if the weight change at 15 min is less than 1%. |
| | | Stage II: increasing humidity by 10% every 150 min until humidity reaches 80%, and proceeding to the next stage if the weight change at 15 min is less than 1%. |
| | | Stage III: reducing humidity by 10% every 150 min until humidity reduces to 0%. |
| Judgment criteria | Deliquescent | Sufficient water is absorbed to form liquid |
| | Extremely hygroscopic | The increase in mass is not less than 15% |
| | Hygroscopic | The increase in mass is less than 15% but not less than 2% |
| | Slightly hygroscopic | The increase in mass is less than 2% but not less than 0.2% |
| | Non-hygroscopic or hardly hygroscopic | The increase in mass is less than 0.2% |

The thermogravimetric analysis result of the crystal form A is shown in FIG. 1d: when the relative humidity is in the range of 0%-80%, the weight change is about 12%, so the crystal form A is hygroscopic.

The thermogravimetric analysis result of the crystal form C is shown in FIG. 2d: when the relative humidity is in the range of 0%-80%, the weight change is about 1.8%, so the crystal form C is slightly hygroscopic.

The thermogravimetric analysis result of the crystal form J is shown in FIG. 3d: when the relative humidity is in the range of 0%-80%, the weight change is about 4.7%, so the crystal form J is hygroscopic.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

Unless otherwise defined, all technical and scientific terms used herein have the same meaning as commonly understood by those skilled in the art. In addition, any methods and materials similar or equivalent to those described herein can be used in the methods of the present invention. Better and preferred embodiments and materials described herein are for illustrative purposes only.

In this specification, unless otherwise specified, a value or a value range, whether preceded by "about" or "approximately" or not, encompasses an approximate range equivalent to the value or the value range as understood by those skilled in the relevant art, e.g., a range of ±10% around the value or end value, or the range of ±5%, ±3%, ±2%, ±1%, or ±0.5% around the value or end value.

The present invention will be further illustrated in detail with reference to the following examples. It is to be understood, however, that these examples are set forth merely for purposes of illustration and are not intended to limit the scope of the present invention.

### Example 1: Preparation of Crystal Form A of Kudinoside A and X-Ray Powder Diffraction Assay Thereof

Specific steps for preparing the crystal form A of Kudinoside A are as follows:
20 kg of *kudingcha* powder was subjected to percolation extraction with 34.4 kg of an 80% ethanol solution and concentrated to obtain an extract in the form of an extractum, and water was added to obtain 17 kg of final extractum; the extractum was diluted with 10.2 kg of anhydrous ethanol and centrifuged to obtain a crude sample.

The crude sample was purified successively by high-pressure chromatography twice using a chromatographic column with 30 µm filler and a chromatographic column with 10 µm filler, respectively, and then decolorized with active carbon to obtain crude powder of Kudinoside A; the crude powder of Kudinoside A was dissolved in 35% ethanol in an 80 °C water bath and then placed in a 6 °C water bath to separate out crystals; the crystals were freeze-dried for 2 h at 0 °C, then dried for 4 h at 25 °C, and finally dried at 35 °C for 6 h; finally, the crystals were crushed to obtain about 170 g of a crystal form of Kudinoside A (namely crystal form A).

The prepared crystal form A was subjected to the X-ray diffraction assay, and the instrument used and parameters thereof are shown in Table 4. The data obtained are shown in Table 1, i.e., characteristic peaks are present at 2θ angles shown in Table 1.

### Example 2: Preparation of Crystal Form C of Kudinoside A and X-Ray Powder Diffraction Assay Thereof

Specific steps for preparing the crystal form C of Kudinoside A are as follows:
a. Firstly, the crystal form A was prepared according to the preparation method shown in Example 1;
b. 1 mL of acetonitrile and 2 mL of isopropyl ether were added to 100 mg of the crystal form A to obtain a suspension, which was stirred for 4 days at room temperature and then centrifuged. A precipitate was dried under vacuum at 30 °C overnight to obtain the crystal form C.

The crystal form C of Kudinoside A was subjected to the X-ray powder diffraction assay, and instrument used and parameters thereof are shown in Table 4. The data obtained are shown in Table 2, i.e., characteristic peaks are present at 2θ angles shown in Table 2.

### Example 3: Preparation of Crystal Form J of Kudinoside A and X-Ray Powder Diffraction Assay Thereof

Specific steps for preparing the crystal form J of Kudinoside A are as follows:
a. Firstly, the crystal form A was prepared according to the preparation method shown in Example 1;
b. 3 mL of saturated aqueous ethyl acetate solution was added to 100 mg of the crystal form A to obtain a suspension, which was stirred for 4 days at room temperature, centrifuged, and dried under vacuum at 30 °C overnight to obtain the crystal form J.

The crystal form J of Kudinoside A was subjected to the X-ray powder diffraction assay, and the instrument used and parameters thereof are shown in Table 4. The data obtained are shown in Table 3, i.e., characteristic peaks are present at 2θ angles shown in Table 3.

### Example 4: Stability Tests of Crystal Form A of Kudinoside A

The crystal form A obtained in Example 1 was subjected to stability tests under the conditions shown in Table 8. The crystal form A was placed in medicine bottles made from low borosilicate glass, and a medicinal composite bag (namely an aluminum foil bag and a small bag of silica gel desiccant) was sleeved outside each bottle, and the bottles were sealed and stored after being vacuumized.

**Table 8. Conditions of Stability Tests**

| Item | Test conditions |
|---|---|
| Accelerated test | Temperature: 40±2 °C; humidity: 75%±5% |
| Long-term test 1 | Temperature: 25±2 °C; humidity: 60%±5% |
| Long-term test 2 | Temperature: 5±3 °C; |

High performance liquid chromatography was used to determine the appearance, moisture, the content of Kudinoside A, and related substances in the crystal form A. The instrument used was an ultra-high performance liquid chromatograph equipped with a DAD (or PDA) detector, and related parameters are as follows:
Chromatographic column: Agilent C18 (2.1*100 mm, 1.8-µm);
Mobile phase A: acetonitrile;
Mobile phase B: ultra-pure water;
Detection wavelength: 225 nm;
Flow rate: 0.6 mL/min;
Injection volume: 5 µL;
Column temperature: 38 °C;
Operating time: 28 min;
Isocratic elution: mobile phase A:mobile phase B = 26:74 (V/V);
Diluent: acetonitrile:water = 30:70 (V/V);
Blank solution: diluent.

### I. Specific steps for determining the content of Kudinoside A are as follows:

**Preparation of an impurity A stock solution:** about 10 mg of an impurity A (namely Kudinoside B) was precisely weighed out, placed in a 100 mL volumetric flask, and dissolved and diluted to a volume indicated by the scale with a diluent, and then the resulting solution was uniformly mixed, thus obtaining a solution containing about 100 µg of impurity A per 1 mL, namely the impurity A stock solution.

**Preparation of an impurity D stock solution:** the steps were the same as those for the preparation of the impurity A stock solution described above, and the impurity A was replaced with the impurity D (namely Kudinoside C), thus obtaining a solution containing about 100 µg of impurity D per 1 mL.

**Preparation of an impurity F stock solution:** the steps were the same as those for the preparation of the impurity A stock solution described above, and the impurity A was replaced with the impurity F (namely Kudinoside D), thus obtaining a solution containing about 100 µg of impurity F per 1 mL.

**Preparation of a resolution solution:** 50 mg of Kudinoside A was precisely weighed out, placed in a 100 mL volumetric flask, and dissolved and diluted to a volume indicated by the scale with a diluent, and then the resulting solution was uniformly mixed; 1 mL of the solution and 0.6 mL of each of the impurity A stock solution, the impurity D stock solution, and the impurity F stock solution were precisely measured out, placed in a 10 mL volumetric flask, and diluted to a volume indicated by the scale with a diluent, and the resulting solution was uniformly mixed, thus obtaining a solution containing 50 µg of Kudinoside A, 6 µg of impurity A, 6 µg of impurity D, and 6 µg of impurity F per 1 mL, which served as the resolution solution.

**Preparation of a standard solution 1:** about 50 mg of a reference substance of Kudinoside A was precisely weighed out, placed in a 100 mL volumetric flask, and dissolved and diluted to a volume indicated by the scale with a diluent, and then the resulting solution was uniformly mixed; 1 mL of the solution was precisely measured out, placed in a 10 mL volumetric flask, and diluted to a volume indicated by the scale with a diluent, and the resulting solution was uniformly mixed, thus obtaining a solution containing about 50 µg of KA per 1 mL, which served as the standard solution 1.

**Preparation of a standard solution 2:** the standard solution 2 was prepared in parallel according to the steps for the preparation of the standard solution 1 described above.

**Preparation of a test sample solution:** about 50 mg of a sample was precisely weighed out, placed in a 100 mL volumetric flask, and dissolved and diluted to a volume indicated by the scale with a diluent, and then the resulting solution was uniformly mixed; 1 mL of the solution was precisely measured out, placed in a 10 mL volumetric flask, and diluted to a volume indicated by the scale with a diluent, and the resulting solution was uniformly mixed, thus obtaining the test sample solution (at a concentration of 50 µg/mL) (the solution was prepared in duplicate).

All sample solutions needed to be filtered through a 0.22 µm PTFE needle filter before UPLC analysis.

**Sample injection procedure:** the blank solution, the resolution solution, the standard solution 1, the standard solution 2, the test sample solution, and the standard solution 1 were each injected according to the chromatographic conditions, and then the needle was withdrawn, and the whole chromatographic process was recorded.

**Calculation of results:** the content of Kudinoside A was calculated using the standard solution 1 through the external standard method.

### II. Specific steps for determining the content of related substances are as follows:

Firstly, an impurity A stock solution, an impurity D stock solution, an impurity F stock solution, a standard solution 1, and a standard solution 2 were prepared according to the method for determining the content of Kudinoside A described above. Next, the following solutions were prepared:
**Resolution solution:** 60 mg of Kudinoside A was precisely weighed out and placed in a 100 mL volumetric flask, 0.9 mL of each of the impurity A stock solution, the impurity D stock solution, and the impurity F stock solution was added, and the mixture was diluted to a volume indicated by the scale with a diluent, and then the resulting solution was uniformly mixed, thus obtaining the resolution solution (Kudinoside A, impurity A, impurity D, and impurity F at a concentration of 600 µg/mL, 0.9 µg/mL, 0.9 µg/mL, and 0.9 µg/mL, respectively).

**Sensitivity solution:** 1 mL of the standard solution 1 was precisely measured out, placed in a 100 mL volumetric flask, and diluted to a volume indicated by the scale with a diluent, and then the resulting solution was uniformly mixed; 3 mL of the solution was precisely measured out, placed in a 100 mL volumetric flask, and diluted to a volume indicated by the scale with a diluent, and the resulting solution was uniformly mixed, thus obtaining a solution containing about 0.15 µg of KA per 1 mL, which served as the sensitivity solution (equivalent to a 0.025% impurity limit solution).

**Test sample solution:** 60 mg of Kudinoside A was precisely weighed out, placed in a 100 mL volumetric flask, and dissolved and diluted to a volume indicated by the scale with a diluent, and then the resulting solution was uniformly mixed, thus obtaining the test sample solution. Two samples were prepared in parallel and named TS1 and TS2.

**Control solution:** 1 mL of the test sample solution was precisely measured out, placed in a 100 mL volumetric flask, and diluted to a volume indicated by the scale with a diluent. 1 mL of the solution was precisely measured out, placed in a 10 mL volumetric flask, and diluted to a volume indicated by the scale with a diluent. Two samples were prepared in parallel and named TSR1 and TSR2.

All sample solutions needed to be filtered through a 0.22 µm PTFE needle filter before UPLC analysis.

**Sample injection procedure:** the blank solution, the resolution solution, the sensitivity solution, the standard solution 1, the standard solution 2, the test sample solution, the control solution, and the standard solution 1 were injected successively for detection according to the chromatographic conditions, and the whole chromatographic process was recorded.

**Calculation of results:** the content of each related substance in the test sample solution was calculated by a self-comparison method with correction factors, wherein the correction factors of the impurity A (i.e., Kudinoside B), the impurity D (i.e., Kudinoside C), and the impurity F (i.e., Kudinoside D) were 1.13, 0.80, and 0.82, respectively.

By using the above method, the stability data of the crystal form A were obtained, which are shown in Tables 9, 10, and 11.

**Table 9. Long-term test 1**

| **Detection items** | | **Limit requirement** | **Test time (month)** | | | | | | | |
|---|---|---|---|---|---|---|---|---|---|---|
| | | | **0** | **1** | **3** | **4** | **6** | **9** | **12** | **24** |
| Appearance | | White powder | White powder | White powder | White powder | White powder | White powder | White powder | White powder | White powder |
| Content of Kudinoside A | | Should be 98.0%-102.0% of the labeled amount | 100.2 | 101.4 | 100.2 | 99.2 | 98.3 | 98.8 | 99.8 | 100.2 |
| Related substances | Content of impurity A (Kudinoside B) | Not more than 0.15% | N.D. | N.D. | N.D. | N.D. | N.D. | N.D. | N.D. | N.D. |
| | Content of impurity D (Kudinoside C) | Not more than 0.15% | N.D. | N.D. | N.D. | N.D. | N.D. | N.D. | N.D. | N.D. |
| | Content of impurity F (Kudinoside D) | Not more than 0.30% | 0.09 | 0.09 | 0.12 | 0.13 | 0.15 | 0.19 | 0.19 | 0.21 |
| | Total content of impurities | Not more than 2.0% | 0.09 | 0.09 | 0.12 | 0.13 | 0.15 | 0.19 | 0.19 | 0.35 |

| | | | | | | | | | | |
|---|---|---|---|---|---|---|---|---|---|---|
| Note: N.D. represents that the result is lower than the detection limit (the detection limits of the impurity A, the impurity D, and the impurity F are 0.03%, 0.02%, 0.05%, and 0.02%, respectively); | | | | | | | | | | |

**Table 10. Long-term test 2**

| **Detection items** | | **Limit requirement** | **Test time (month)** | | | | | | | |
|---|---|---|---|---|---|---|---|---|---|---|
| | | | **0** | **1** | **3** | **4** | **6** | **9** | **12** | **24** |
| Appearance | | White powder | White powder | White powder | White powder | White powder | White powder | White powder | White powder | White powder |
| Content of Kudinoside A | | Should be 98.0%-102.0% of the labeled amount | 100.2 | 101.8 | 99.9 | 99.8 | 98.6 | 99.2 | 99.8 | 101.3 |
| Related substances | Content of impurity A (Kudinoside B) | Not more than 0.15% | N.D. | N.D. | N.D. | N.D. | N.D. | N.D. | N.D. | N.D. |
| | Content of impurity D (Kudinoside C) | Not more than 0.15% | N.D. | N.D. | N.D. | N.D. | N.D. | N.D. | N.D. | N.D. |
| | Content of impurity F (Kudinoside D) | Not more than 0.30% | 0.09 | 0.08 | 0.10 | 0.08 | 0.09 | 0.09 | 0.09 | 0.08 |
| | Total content of impurities | Not more than 2.0% | 0.09 | 0.08 | 0.10 | 0.08 | 0.09 | 0.09 | 0.09 | 0.08 |

| | | | | | | | | | | |
|---|---|---|---|---|---|---|---|---|---|---|
| Note: N.D. represents that the result is lower than the detection limit (the detection limits of the impurity A, the impurity D, and the impurity F are 0.03%, 0.02%, 0.05%, and 0.02%, respectively); | | | | | | | | | | |

**Table 11. Accelerated test**

| **Detection items** | | **Limit requirement** | **Test time (month)** | | | | | |
|---|---|---|---|---|---|---|---|---|
| | | | **0** | **1** | **3** | **4** | **5** | **6** |
| Appearance | | White powder | White powder | White powder | White powder | White powder | White powder | White powder |
| Content of Kudinoside A | | Should be 98.0%-102.0% of the labeled amount | 100.2 | 101.7 | 99.0 | 98.0 | 98.9 | 98.4 |
| Related substances | Content of impurity A | Not more than 0.15% | N.D. | N.D. | N.D. | N.D. | N.D. | N.D. |
| | (Kudinoside B) | | | | | | | |
| | Content of impurity D (Kudinoside C) | Not more than 0.15% | N.D. | N.D. | N.D. | N.D. | N.D. | N.D. |
| | Content of impurity F (Kudinoside D) | Not more than 0.30% | 0.09 | 0.17 | 0.26 | 0.29 | 0.29 | 0.29 |
| | Total content of impurities | Not more than 2.0% | 0.09 | 0.17 | 0.43 | 0.52 | 0.60 | 0.66 |

| | | | | | | | | |
|---|---|---|---|---|---|---|---|---|
| Note: N.D. represents that the result is lower than the detection limit (the detection limits of the impurity A, the impurity D, and the impurity F are 0.03%, 0.02%, 0.05%, and 0.02%, respectively); | | | | | | | | |

It can be seen from the results shown in Tables 9-11 that the crystal form A obtained in Example 1 met the quality standards specified under any experimental conditions, which indicates that the crystal form A has good stability.

### Example 5: Hygroscopicity Tests of Crystal Forms A, C, and J of Kudinoside A

About 10 mg of each of the crystal forms A, C, and J obtained in Examples 1-3 was tested for its hygroscopicity using a dynamic vapor sorption (DVS) instrument (test conditions: a temperature of 25 °C and a relative humidity of 0%-80%), and the hygroscopicity of each crystal form was judged using the criteria shown in Table 12 below. For the steps and the judgment criteria of the hygroscopicity test, reference is made to the General Requirement 9103-Guidelines for Hygroscopicity in Pharmacopoeia of the People's Republic of China 2020 (Volume IV).

**Table 12. Judgment criteria for hygroscopicity**

| Degree of hygroscopicity | Limits of increase in mass |
|---|---|
| Deliquescent | Sufficient water is absorbed to form liquid |
| Extremely hygroscopic | The increase in mass is not less than 15% |
| Hygroscopic | The increase in mass is less than 15% but not less than 2% |
| Slightly hygroscopic | The increase in mass is less than 2% but not less than 0.2% |
| Non-hygroscopic or hardly hygroscopic | The increase in mass is less than 0.2% |

In this experiment, the dynamic vapor sorption (DVS) graphs of the crystal forms A, C, and J are shown in FIGs. 1d, 2d, and 3d, respectively. Also, the results of their hygroscopicity are detailed in Table 13 below.

**Table 13. Results of hygroscopicity tests of crystal forms A, C, and J**

| Crystal form | Increase in mass (relative humidity: 0%-80%) | Degree of hygroscopicity |
|---|---|---|
| A | 12% | Hygroscopic |
| C | 1.8% | Slightly hygroscopic |
| J | 4.7% | Hygroscopic |

As can be seen from Table 13: the hygroscopicity of the crystal forms A, C and J is as follows: A > J > C. According to the hygroscopicity criteria in the 2020 edition, the crystal form C is slightly hygroscopicity, and the crystal forms A and J are hygroscopic. The crystal form C has significant advantages in hygroscopicity.

### Example 6: Stability Test of Crystal Form J of Kudinoside A

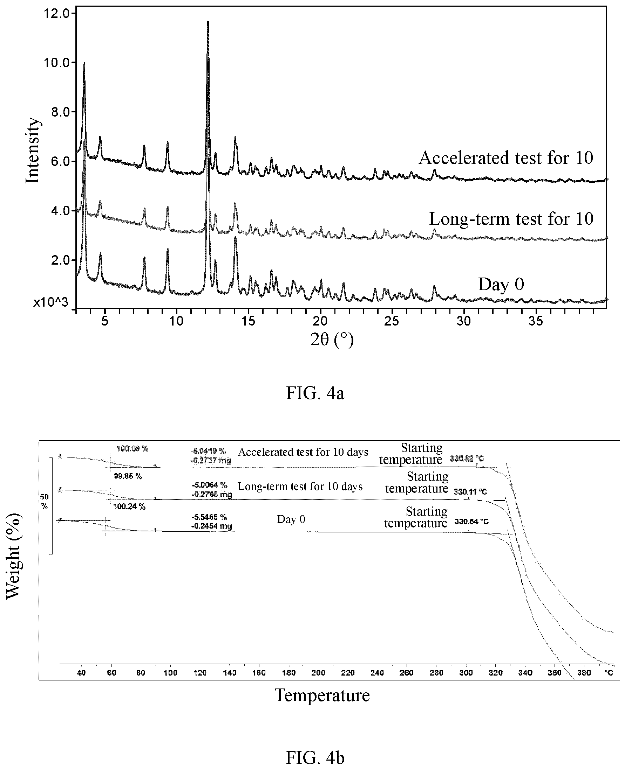

25 mg of the crystal form J was spread in a culture dish and left to stand, with the culture dish uncovered, in the dark for 10 days under long-term conditions (temperature: 25±2 °C; relative humidity: 65%±10%) and acceleration conditions (temperature: 40±2 °C; relative humidity: 75%±10%), and then the crystal form J was subjected to X-ray powder diffraction assay and thermogravimetric analysis. The X-ray powder diffractometer used and parameters thereof are shown in Table 4; the thermogravimetric analyzer used and parameters thereof are shown in Table 6. The assay results are shown in FIG. 4.

As can be seen from FIG. 4a, under the conditions of the long-term test and the accelerated test, the characteristic peaks on the X-ray powder diffraction pattern of the crystal form J were not changed, which proved that the crystal form was not changed; As can be seen from FIG. 4b, under the conditions of the long-term test and the accelerated test, the weight loss condition of the crystal form J was not changed. In conclusion, the crystal form J has good stability.

### Example 7: Competition Experiment of Crystal Form A and Crystal Form J of Kudinoside A

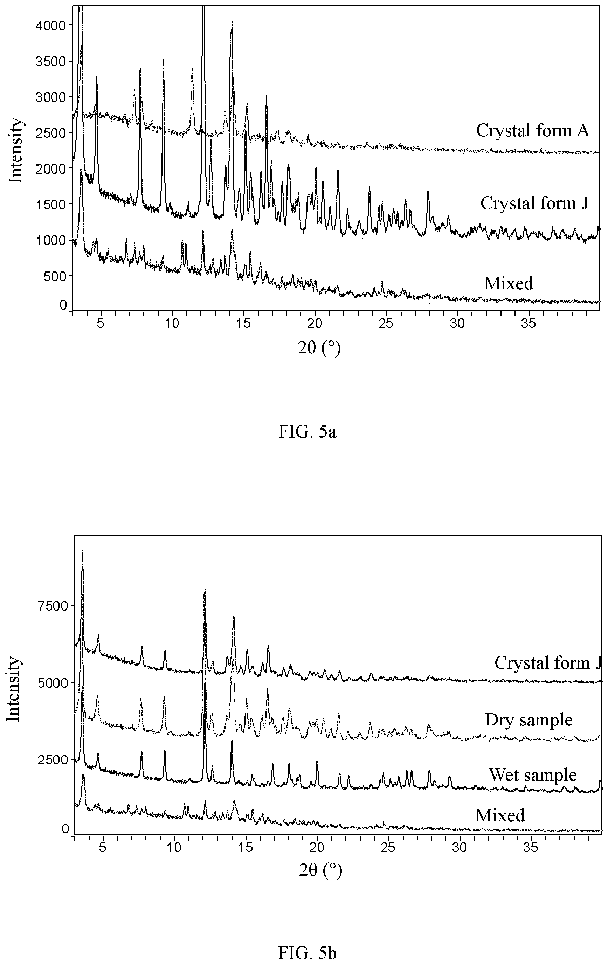

Firstly, an initial mixing sample was prepared: samples of the crystal form A and the crystal form J of Kudinoside A with the equivalent quantity were uniformly mixed, and sampling was carried out for XRPD detection. The instrument used and parameters thereof are shown in Table 4, and the resulting comparison graph is shown in FIG. 5a.

Water was added to the sample to form a suspension, which was stirred at room temperature and then centrifuged 3 days later, and sampling was carried out for XRPD detection (wet sample); after drying under vacuum at 30 °C, the sample was subjected to XRPD detection (dry sample). The instrument used and parameters thereof are shown in Table 4, and the detection results are shown in FIG. 5b.

As can be seen from FIG. 5b, the resulting hydrate was determined to be the crystal form J whether a dry sample or a wet sample was used. Therefore, it can be seen that under certain conditions, the crystal form A tends to convert to the crystal form J.

### Example 8: Preparation of Other New Crystal Forms of Kudinoside A and X-Ray Powder Diffraction Assays Thereof

### 1. Preparation of crystal form B and X-ray powder diffraction assay thereof

Preparation method of crystal form B of Kudinoside A: firstly, the crystal form A was prepared according to the preparation method shown in Example 1; a proper amount of the crystal form A sample was heated to 120 °C on a heat stage and subjected to heat preservation for 5 min, thus obtaining the crystal form B.

The crystal form B prepared above was subjected to X-ray diffraction assay. The instrument used and parameters thereof are shown in Table 4, and the resulting data are shown in Table 14:

**Table 14. List of X-ray powder diffraction peaks of crystal form B**

| **2Θ°** | **d-spacing** | **Relative intensity (%)** |
|---|---|---|
| 3.684 | 23.9619 | 5.5 |
| 4.822 | 18.3124 | 29.6 |
| 5.221 | 16.9132 | 4.1 |
| 6.043 | 14.613 | 4.1 |
| 6.765 | 13.0557 | 11.5 |
| 7.441 | 11.8709 | 100 |
| 7.878 | 11.2127 | 61.5 |
| 8.104 | 10.9015 | 17.6 |
| 8.804 | 10.0352 | 10.7 |
| 9.723 | 9.0891 | 11.7 |
| 11.842 | 7.4668 | 73.4 |
| 12.224 | 7.2348 | 15.6 |
| 12.525 | 7.0613 | 30 |
| 14.045 | 6.3003 | 16 |
| 14.305 | 6.1866 | 48.1 |
| 14.606 | 6.0596 | 17.5 |
| 15.088 | 5.8672 | 7.9 |
| 15.409 | 5.7458 | 28 |
| 15.865 | 5.5816 | 10.6 |
| 17.065 | 5.1915 | 7.2 |
| 17.825 | 4.972 | 8.8 |
| 18.185 | 4.8743 | 8.6 |
| 18.595 | 4.7678 | 6.6 |
| 19.008 | 4.6651 | 8.9 |
| 19.186 | 4.6222 | 11 |
| 20.006 | 4.4346 | 6.6 |
| 21.231 | 4.1813 | 4.3 |
| 23.892 | 3.7213 | 4.4 |
| 24.673 | 3.6053 | 4.4 |
| 24.901 | 3.5728 | 4 |
| 29.572 | 3.0182 | 4.2 |

The crystal form B has characteristic peaks at 2θ angles described in Table 14 above. In addition, the crystal form B can only exist stably at high temperature.

### 2. Preparation of crystal form D and X-ray powder diffraction assay thereof

15 mL of isopropanol was added to 100 mg of amorphous Kudinoside A, and then the mixture was subjected to sonication and stirred in a water bath at 60 °C until the Kudinoside A was completely dissolved; then a solid was slowly separated out, and the mixture was slowly cooled to room temperature, stirred for 4 days, and then centrifuged, thus obtaining the crystal form D. The crystal form D prepared above was subjected to X-ray diffraction assay. The instrument used and parameters thereof are shown in Table 4, and the resulting data are shown in Table 15:

**Table 15. List of X-ray powder diffraction peaks of crystal form D**

| **2Θ°** | **d-spacing** | **Relative intensity (%)** |
|---|---|---|
| 4.505 | 19.5974 | 5.9 |
| 6.225 | 14.1873 | 14.5 |
| 7.184 | 12.2955 | 5.1 |
| 8.151 | 10.8384 | 1.1 |
| 9.867 | 8.9571 | 10.6 |
| 12.441 | 7.1088 | 100 |
| 12.86 | 6.8782 | 4.3 |
| 13.447 | 6.5792 | 3.3 |
| 14.012 | 6.3153 | 2.2 |
| 14.825 | 5.9706 | 0.8 |
| 15.145 | 5.8453 | 2 |
| 15.887 | 5.5737 | 1.2 |
| 16.65 | 5.32 | 2.8 |
| 16.86 | 5.2542 | 1.6 |
| 17.601 | 5.0346 | 1.1 |
| 17.93 | 4.943 | 1 |
| 18.346 | 4.832 | 2.6 |
| 18.667 | 4.7496 | 0.9 |
| 18.965 | 4.6755 | 5 |
| 19.432 | 4.5641 | 0.8 |
| 19.8 | 4.4802 | 1.2 |
| 20.782 | 4.2707 | 0.8 |
| 21.569 | 4.1167 | 1 |
| 22.049 | 4.0281 | 2.9 |
| 22.778 | 3.9007 | 0.8 |
| 23.284 | 3.8172 | 1 |
| 23.949 | 3.7126 | 2 |
| 24.472 | 3.6344 | 1.2 |
| 25.133 | 3.5404 | 4.1 |
| 26.831 | 3.32 | 12 |
| 27.06 | 3.2924 | 1 |
| 27.569 | 3.2328 | 0.7 |
| 28.33 | 3.1477 | 1.1 |
| 28.65 | 3.1133 | 0.9 |
| 29.866 | 2.9892 | 0.7 |
| 30.078 | 2.9686 | 12 |
| 32.477 | 2.7546 | 0.6 |
| 32.85 | 2.7241 | 0.6 |
| 33.938 | 2.6392 | 0.8 |
| 34.29 | 2.613 | 0.6 |
| 36.769 | 2.4423 | 0.6 |
| 39.516 | 2.2786 | 0.9 |

The crystal form D has characteristic peaks at 2θ angles described in Table 15 above. In addition, the crystal form D can only exist stably when the sample is wet.

### 3. Preparation of crystal form E and X-ray powder diffraction assay thereof

Preparation method of crystal form E of Kudinoside A: 0.8 mL of ethanol and 0.2 mL of water were added to 30 mg of amorphous Kudinoside A, and then the mixture was heated in a water bath at 70 °C until the Kudinoside A was completely dissolved; then the solution was slowly cooled to room temperature and left to stand overnight, and a solid was separated out, thus obtaining the crystal form E.

The crystal form E prepared above was subjected to X-ray diffraction assay. The instrument used and parameters thereof are shown in Table 4, and the resulting data are shown in Table 16:

**Table 16. List of X-ray powder diffraction peaks of crystal form E**

| **2Θ°** | **d-spacing** | **Relative intensity (%)** |
|---|---|---|
| 3.574 | 24.7011 | 1.3 |
| 4.541 | 19.4413 | 6.9 |
| 5.577 | 15.832 | 8.6 |
| 7.667 | 11.5208 | 3 |
| 9.329 | 9.4725 | 0.6 |
| 10.82 | 8.1702 | 0.5 |
| 11.137 | 7.9381 | 100 |
| 11.699 | 7.5582 | 1 |
| 12.145 | 7.2813 | 2 |
| 13.242 | 6.6806 | 0.5 |
| 13.588 | 6.5114 | 3.9 |
| 14.022 | 6.3107 | 0.7 |
| 14.37 | 6.1588 | 17.8 |
| 14.89 | 5.9446 | 0.4 |
| 15.454 | 5.7289 | 0.6 |
| 17.594 | 5.0368 | 0.6 |
| 18.148 | 4.8843 | 2.4 |
| 18.746 | 4.7296 | 1 |
| 19.846 | 4.4699 | 2.3 |
| 20.807 | 4.2655 | 2.8 |
| 21.106 | 4.2059 | 0.7 |
| 21.671 | 4.0975 | 0.4 |
| 22.328 | 3.9784 | 1.3 |
| 23.476 | 3.7863 | 0.7 |
| 24.269 | 3.6644 | 0.7 |
| 24.798 | 3.5873 | 0.5 |
| 25.43 | 3.4996 | 2.3 |
| 25.692 | 3.4646 | 1 |
| 26.19 | 3.3997 | 1.4 |
| 26.596 | 3.3488 | 0.3 |
| 27.411 | 3.251 | 0.6 |
| 28.013 | 3.1826 | 3.8 |
| 28.251 | 3.1563 | 1.5 |
| 28.738 | 3.1038 | 1.7 |
| 28.932 | 3.0835 | 1.1 |
| 30.089 | 2.9675 | 0.3 |
| 31.093 | 2.8739 | 1.7 |
| 31.992 | 2.7952 | 0.5 |
| 33.418 | 2.6791 | 1.1 |
| 33.956 | 2.6379 | 1 |
| 34.169 | 2.622 | 0.5 |
| 34.534 | 2.595 | 0.7 |
| 38.06 | 2.3624 | 0.2 |
| 38.47 | 2.3381 | 0.4 |
| 39.604 | 2.2737 | 0.3 |

The crystal form E has characteristic peaks at 2θ angles described in Table 16 above. In addition, the crystal form E can only exist stably when the sample is wet.

### 4. Preparation of crystal form G and X-ray powder diffraction assay thereof

Preparation method of crystal form G of Kudinoside A: 3 mL of tetrahydrofuran and 1.2 mL of water were added to 100 mg of amorphous Kudinoside A, and the mixture was subjected to sonication to completely dissolve the Kudinoside A, and the resulting solution was left to stand at room temperature to volatilize with the container uncovered, thus obtaining the crystal form G.

The crystal form G prepared above was subjected to X-ray diffraction assay. The instrument used and parameters thereof are shown in Table 4, and the resulting data are shown in Table 17:

**Table 17. List of X-ray powder diffraction peaks of crystal form G**

| **2Θ°** | **d-spacing** | **Relative intensity (%)** |
|---|---|---|
| 3.36 | 26.2771 | 100 |
| 5.682 | 15.5402 | 27.7 |
| 7.346 | 12.0233 | 15.9 |
| 7.781 | 11.3533 | 42.3 |
| 9.187 | 9.6179 | 37.5 |
| 9.943 | 8.8886 | 46.3 |
| 10.917 | 8.0975 | 7.9 |
| 11.442 | 7.7271 | 12.8 |
| 11.967 | 7.3893 | 16.8 |
| 12.547 | 7.0489 | 4.3 |
| 13.381 | 6.6116 | 6.3 |
| 13.726 | 6.4461 | 10 |
| 14.424 | 6.1359 | 23.6 |
| 14.812 | 5.9756 | 8.5 |
| 15.181 | 5.8315 | 5.6 |
| 16.459 | 5.3813 | 6.9 |
| 17.009 | 5.2086 | 19.2 |
| 17.504 | 5.0623 | 40.1 |
| 18.447 | 4.8056 | 23.8 |
| 18.788 | 4.7191 | 15.1 |
| 19.766 | 4.4879 | 64.9 |
| 20.027 | 4.43 | 36.1 |
| 20.468 | 4.3355 | 13.8 |
| 21.424 | 4.1441 | 12.4 |
| 22.488 | 3.9504 | 15.6 |
| 22.828 | 3.8924 | 13.6 |
| 23.328 | 3.81 | 6.5 |
| 24.108 | 3.6885 | 12.1 |
| 25.55 | 3.4835 | 17.1 |
| 25.951 | 3.4306 | 22.2 |
| 26.169 | 3.4025 | 18.5 |
| 27.59 | 3.2303 | 9.1 |
| 28.57 | 3.1218 | 30.3 |
| 29.017 | 3.0747 | 6.4 |
| 29.857 | 2.9901 | 3.5 |
| 30.331 | 2.9444 | 10.2 |
| 31.534 | 2.8347 | 7.8 |
| 32.052 | 2.7902 | 3.3 |
| 32.99 | 2.7129 | 5.3 |
| 33.394 | 2.681 | 7.3 |
| 33.712 | 2.6564 | 8.1 |
| 34.254 | 2.6156 | 13 |
| 34.955 | 2.5648 | 10.1 |
| 35.373 | 2.5354 | 11.3 |
| 36.129 | 2.4841 | 3.2 |
| 37.289 | 2.4094 | 5 |
| 37.794 | 2.3784 | 20.3 |
| 38.311 | 2.3475 | 7.6 |

The crystal form G has characteristic peaks at 2θ angles described in Table 17 above. In addition, the crystal form G can only exist stably when the sample is wet.

### 5. Preparation of crystal form I₁ and X-ray powder diffraction assay thereof

Preparation method of crystal form I₁ of Kudinoside A: 1 mL of trifluoroethanol was added to 30 mg of amorphous Kudinoside A, and the mixture was subjected to sonication to completely dissolve the Kudinoside A, and the resulting solution was left to stand at room temperature to volatilize to dryness with the container uncovered, thus obtaining the crystal form I₁.

The crystal form I₁ prepared above was subjected to X-ray diffraction assay. The instrument used and parameters thereof are shown in Table 4, and the resulting data are shown in Table 18:

**Table 18. List of X-ray powder diffraction peaks of crystal form I₁**

| **2Θ°** | **d-spacing** | **Relative intensity (%)** |
|---|---|---|
| 5.108 | 17.2868 | 100 |
| 6.406 | 13.7853 | 94.3 |
| 7.684 | 11.4952 | 36.6 |
| 12.82 | 6.8993 | 38.5 |
| 13.998 | 6.3212 | 34 |
| 14.329 | 6.1761 | 29.4 |
| 14.705 | 6.0191 | 33.2 |
| 16.091 | 5.5036 | 26.4 |
| 19.227 | 4.6123 | 27.2 |
| 20.592 | 4.3097 | 18.9 |
| 25.806 | 3.4495 | 16.6 |

The crystal form I₁ has characteristic peaks at 2θ angles described in Table 18 above.

### 6. Preparation of crystal form I₂ and X-ray powder diffraction assay thereof

Preparation method of crystal form I₂ of Kudinoside A: 3 mL of trifluoroethanol was added to 100 mg of amorphous Kudinoside A, and the mixture was subjected to sonication to completely dissolve the Kudinoside A, and the resulting solution was filtered and left to stand at room temperature to volatilize to dryness with the container uncovered, thus obtaining the crystal form I₂.

The crystal form I₂ prepared above was subjected to X-ray diffraction assay. The instrument used and parameters thereof are shown in Table 4, and the resulting data are shown in Table 19:

**Table 19. List of X-ray oowder diffraction peaks of crystal form I₂**

| **2Θ°** | **d-spacing** | **Relative intensity (%)** |
|---|---|---|
| 5.525 | 15.9834 | 16.4 |
| 6.556 | 13.4717 | 18.7 |
| 7.937 | 11.1296 | 30.7 |
| 12.202 | 7.2476 | 16.9 |
| 13.848 | 6.3897 | 99.2 |
| 14.083 | 6.2837 | 100 |
| 15.604 | 5.6742 | 18.6 |
| 28.891 | 3.0878 | 10.2 |

The crystal form I₂ has characteristic peaks at 2θ angles described in Table 19 above.

### 7. Preparation of crystal form K and X-ray powder diffraction assay thereof

Preparation method of crystal form K of Kudinoside A: 15 mL of ethanol was added to 100 mg of amorphous Kudinoside A, and the mixture was subjected to sonication to completely dissolve the Kudinoside A, and the resulting solution was filtered and concentrated under reduced pressure at 28 °C, thus obtaining the crystal form K.

The crystal form K prepared above was subjected to X-ray diffraction assay. The instrument used and parameters thereof are shown in Table 4, and the resulting data are shown in Table 20:

**Table 20. List of X-ray powder diffraction peaks of crystal form K**

| **2Θ°** | **d-spacing** | **Relative intensity (%)** |
|---|---|---|
| 4.48 | 19.7084 | 26.1 |
| 4.957 | 17.8136 | 5.8 |
| 5.537 | 15.9474 | 8.5 |
| 6.04 | 14.6212 | 4.4 |
| 7.603 | 11.6176 | 25.2 |
| 8.182 | 10.7975 | 9.2 |
| 8.982 | 9.8372 | 0.9 |
| 9.387 | 9.4139 | 2.1 |
| 10.158 | 8.7007 | 1.7 |
| 11.102 | 7.9632 | 91 |
| 11.666 | 7.5792 | 4.9 |
| 12.123 | 7.2948 | 100 |
| 13.544 | 6.5324 | 9.4 |
| 14.345 | 6.1694 | 20.2 |
| 15.285 | 5.792 | 4.2 |
| 15.989 | 5.5384 | 2.1 |
| 16.439 | 5.3878 | 1.6 |
| 17.553 | 5.0485 | 1.5 |
| 18.107 | 4.8951 | 6 |
| 18.705 | 4.74 | 5.5 |
| 19.83 | 4.4735 | 3.1 |
| 20.789 | 4.2693 | 2.8 |
| 21.103 | 4.2064 | 1.4 |
| 21.629 | 4.1053 | 1.4 |
| 22.133 | 4.0129 | 0.8 |
| 22.324 | 3.9791 | 1.5 |
| 23.081 | 3.8502 | 0.7 |
| 23.525 | 3.7786 | 1.6 |
| 23.973 | 3.709 | 1.5 |
| 24.348 | 3.6526 | 3 |
| 25.418 | 3.5013 | 1.6 |
| 25.721 | 3.4608 | 2 |
| 26.208 | 3.3975 | 1.4 |
| 27.169 | 3.2795 | 0.8 |
| 27.491 | 3.2418 | 2.2 |
| 28.051 | 3.1784 | 1.5 |
| 28.289 | 3.1522 | 0.6 |
| 28.968 | 3.0798 | 2.1 |
| 29.956 | 2.9804 | 0.8 |
| 30.374 | 2.9403 | 0.6 |
| 31.207 | 2.8638 | 1.2 |
| 32.035 | 2.7916 | 0.7 |
| 33.433 | 2.6779 | 1.3 |
| 34.134 | 2.6246 | 1.1 |
| 34.429 | 2.6027 | 1.1 |
| 34.594 | 2.5907 | 0.6 |
| 35.915 | 2.4984 | 0.5 |
| 36.751 | 2.4435 | 0.6 |
| 37.968 | 2.3679 | 0.5 |

The crystal form K has characteristic peaks at 2θ angles described in Table 20 above.

### 8. Preparation of crystal form L and X-ray powder diffraction assay thereof

Preparation method of crystal form L of Kudinoside A: firstly, the crystal form J was prepared according to the preparation method shown in Example 3; a proper amount of the crystal form J was heated to 120 °C and subjected to heat preservation for 5 min, thus obtaining the crystal form L.

The crystal form L prepared above was subjected to X-ray diffraction assay. The instrument used and parameters thereof are shown in Table 4, and the resulting data are shown in Table 21:

**Table 21. List of X-ray powder diffraction peaks of crystal form L**

| **2Θ°** | **d-spacing** | **Relative intensity (%)** |
|---|---|---|
| 3.659 | 24.1299 | 13.9 |
| 4.76 | 18.5477 | 9 |
| 6.159 | 14.3378 | 1.8 |
| 7.902 | 11.179 | 11.6 |
| 9.541 | 9.2621 | 21.9 |
| 11.359 | 7.7833 | 2 |
| 12.284 | 7.1992 | 100 |
| 12.822 | 6.8983 | 3.8 |
| 13.604 | 6.5039 | 64.6 |
| 14.585 | 6.0682 | 48 |
| 15.424 | 5.7401 | 35 |
| 16.165 | 5.4784 | 41 |
| 16.926 | 5.2341 | 17.1 |
| 17.344 | 5.1086 | 17.5 |
| 18.165 | 4.8797 | 8.9 |
| 18.806 | 4.7147 | 17.3 |
| 19.348 | 4.5839 | 6.1 |
| 19.969 | 4.4427 | 4 |
| 20.426 | 4.3442 | 12.9 |
| 22.266 | 3.9893 | 4.6 |
| 22.829 | 3.8921 | 2.8 |
| 23.546 | 3.7753 | 6.2 |
| 24.767 | 3.5918 | 3.9 |
| 26.07 | 3.4152 | 6.6 |
| 26.547 | 3.3549 | 2.5 |
| 28.629 | 3.1155 | 4.7 |

The crystal form L has characteristic peaks at 2θ angles described in Table 21 above. In addition, the crystal form L can only exist stably at high temperature.

### Example 9: Biological Activity of Kudinoside A

### 1. Relaxation effect of Kudinoside A on bronchial smooth muscle

Firstly, Kudinoside A solutions with different concentrations were prepared: 0.3253g of the crystal form A of the Kudinoside A obtained in Example 1 was dissolved in 1 mL of dimethyl sulfoxide (DMSO) to prepare a stock solution of the crystal form A of Kudinoside A, and then the stock solution was diluted in sequence with DMSO into solutions of the crystal form A of Kudinoside A at concentrations of 1µM, 3.2µM, 10µM, and 32µM.

Then, bronchial rings were prepared: C57BL/6J mice aged 6 weeks were killed by breaking the neck, left and right bronchi were taken out and subjected to bronchia dissection under a stereomicroscope, and the obtained left and right bronchi were cut into bronchial rings with a length of about 2 mm.

Furthermore, the tension of the bronchial ring was balanced: the bronchial ring was fixed with two metal wires and suspended in a water bath at 37 °C containing 5 mL of balanced salt solution (Kreb solution), and a mixed gas containing 5% of carbon dioxide and 95% of oxygen was continuously introduced; then the tension of the bronchial ring was balanced using a tension transducer and re-balanced after pre-stimulation with 60 mM potassium chloride.

Finally, determination of the airway tension and drug administration were performed: the instrument was calibrated, and the detection system was zeroed; a tension value A was recorded when the tension of the mouse was stable; 5 µL of 10 mM melanin-concentrating hormone was added to 5 mL of Kreb solution to make the final concentration to be 10 µM, and 5 µL of each of the solutions of the crystal form A of Kudinoside A at different concentrations (1 µM, 3.2 µM, 10 µM, and 32 µM) was added separately when the constriction tension of the bronchial ring reached the peak; the change of the bronchial tension was observed and recorded, and the percentage of the airway tension relaxation was calculated, as shown in Table 22. The average tension value 1 minute before adding the solution of Kudinoside A is recorded as B, the average tension value 20 minutes after adding Kudinoside A is recorded as C, and the percentage of airway tension relaxation is (B-C)/(B-A).

**Table 22. Percentages of tension relaxation of bronchial smooth muscle treated with Kudinoside A at different concentrations**

| **Concentration of Kudinoside A** | **Average percentage of tension relaxation** |
|---|---|
| 1 µM | -3.57% |
| 3.2 µM | 21.40% |
| 10 µM | 42.93% |
| 32 µM | 50.09% |

As can be seen from Table 22 above, when the concentration of Kudinoside A is 1 µM, Kudinoside A has no relaxation effect on bronchial smooth muscle; when the concentration of Kudinoside A is 3.2 µM, 10 µM, and 32 µM, the bronchial smooth muscle is relaxed to a certain degree, and the higher the concentration of KA is, the stronger the relaxation effect on the bronchial smooth muscle is.

### 2. Anti-inflammatory effect of Kudinoside A

Firstly, Kudinoside A solutions at different concentrations were prepared, and specific steps were as follows:
(1) Preparation of 15 mg/mL stock solution of the crystal form A of Kudinoside A: 0.50990 g of the crystal form A of Kudinoside A obtained in Example 1 was dissolved in 33.993 mL of propylene glycol under sonication to obtain about 40 mL of 15 mg/mL stock solution of the crystal form A of Kudinoside A, which was stored at room temperature in the dark;
(2) Preparation of 1.5 mg/mL Kudinoside A solution: 2 mL of the 15 mg/mL stock solution of the crystal form A of Kudinoside A was measured out and diluted with 18 mL of 25% propylene glycol to obtain 20 mL of 1.5 mg/mL Kudinoside A solution, and the solution was freshly prepared before use.
(3) Preparation of 0.3 mg/mL Kudinoside A solution: 4 mL of the 1.5 mg/mL Kudinoside A solution was measured out and diluted with 16 mL of 32.5% propylene glycol to obtain 20 mL of 0.3 mg/mL Kudinoside A solution, and the solution was freshly prepared before use.
(4) Preparation of 0.06 mg/mL Kudinoside A solution: 3 mL of the 0.3 mg/mL Kudinoside A solution was measured out and diluted with 12 mL of 32.5% propylene glycol to obtain 15 mL of 0.3 mg/mL Kudinoside A solution, and the solution was freshly prepared before use.

Then, asthma mouse models with obvious pulmonary inflammation were constructed: 100 µL of 6 mg/mL chicken ovalbumin and 4 mg of aluminum hydroxide were injected intraperitoneally to Balb/c mice on day 1, 100 µL of 3.6 mg/mL chicken ovalbumin and 4 mg of aluminum hydroxide were injected on day 8, 100 µL of 1 mg/mL chicken ovalbumin and 4 mg of aluminum hydroxide were injected on day 15, and 6 mL of 2% chicken ovalbumin was administered by nebulization for exciting of 30 min every day from day 20 to day 23, thus constructing asthma mouse models with obvious pulmonary inflammation. For the mice in the blank control group, 100 µL of PBS was used instead of chicken ovalbumin.

Finally, the nebulization treatment was performed: from day 24, the Kudinoside A solutions at different concentrations (0.06 mg/mL, 0.3 mg/mL, and 1.5 mg/mL) were each administered by nebulization twice daily (30 min for each time, 12 mL in total) for treatment, and 6 mL of 2% chicken ovalbumin was administered by nebulization for exciting of 30 min 1 h after the nebulization treatment, and the administration and excitation were performed for 15 days according to this scheme; sampling was carried out 24 h after the last administration and excitation experiment, i.e., on day 39, and the Kudinoside A solution at the corresponding nebulization treatment concentration was administered twice (30 min for each time, 12 mL in total) for treatment before the sampling. The above was the scheme for the experimental groups of the nebulization treatment with Kudinoside A at different doses, and the mice in the negative control group were administered with 30% propylene glycol instead of Kudinoside A for nebulization.

Flow cytometry was adopted to detect proportion of eosinophilic granulocytes (EOs) in white blood cells of mouse alveolar lavage fluid samples. The blank group had 15 mice, the negative control group had 12 mice, and the high-, medium-, and low-concentration Kudinoside A experimental groups had 18 mice, 13 mice, and 14 mice, respectively. The results are shown in Table 23 below.

**Table 23. Results of proportion of eosinophilic granulocytes (EOs)**

| | Blank control group | Negative control group | Kudinoside A group (0.06 mg/mL) | Kudinoside A group (0.3 mg/mL) | Kudinoside A group (1.5 mg/mL) |
|---|---|---|---|---|---|
| Proportion of eosinophilic granulocytes (EOs) | 4.16 | 17.05 | 10.14 | 9.56 | 10.81 |

As can be seen from the results in Table 23, compared with the blank control group, the proportion of eosinophilic granulocytes in the negative control group significantly increases, which indicates that the asthma mouse models of pulmonary inflammation were successfully constructed; compared with the negative control group, the proportion of eosinophilic granulocytes in the Kudinoside A administration group at each dose decreases, which indicates that the Kudinoside A has certain anti-inflammatory effects.

While specific examples of the present invention have been described, it will be apparent to those skilled in the art that various changes and modifications can be made to the present invention without departing from the spirit and scope of the present invention. Therefore, the appended claims encompass all such changes and modifications that fall within the scope of the present invention.

## Claims

1. A crystal form of a Kudinoside A compound, selected from crystal forms A, C, J, B, D, E, G, K, and L, wherein,
the crystal form A has an X-ray powder diffraction (XRPD) pattern comprising characteristic peaks at 2θ angles of 6.7°±0.2°, 7.3°±0.2°, 7.9°±0.2°, 10.5°±0.2°, 10.9°±0.2°, and 14.1°±0.2°;
the crystal form C has an X-ray powder diffraction (XRPD) pattern comprising characteristic peaks at 2θ angles of 7.8°±0.2°, 12.7°±0.2°, 14.4°±0.2°, 14.9°±0.2°, 16.3°±0.2°, 18.6°±0.2°, and 25.1°±0.2°;
the crystal form J has an X-ray powder diffraction (XRPD) pattern comprising characteristic peaks at 2θ angles of 7.7°±0.2°, 9.3°±0.2°, 12.1°±0.2°, 14.1°±0.2°, 15.1°±0.2°, and 16.6°±0.2°;
the crystal form B has an X-ray powder diffraction (XRPD) pattern comprising characteristic peaks at 2θ angles of 7.4°±0.2°, 7.9°±0.2°, 11.8°±0.2°, 12.5°±0.2°, 14.3°±0.2°, and 15.4°±0.2°;
the crystal form D has an X-ray powder diffraction (XRPD) pattern comprising characteristic peaks at 2θ angles of 6.2°±0.2°, 9.9°±0.2°, and 12.4°±0.2°;
the crystal form E has an X-ray powder diffraction (XRPD) pattern comprising characteristic peaks at 2θ angles of 5.6°±0.2°, 11.1°±0.2°, and 14.4°±0.2°;
the crystal form G has an X-ray powder diffraction (XRPD) pattern comprising characteristic peaks at 2θ angles of 5.7°±0.2°, 7.8°±0.2°, 9.2°±0.2°, 9.9°±0.2°, 17.5°±0.2°, 19.8°±0.2°, 20.0°±0.2°, 28.5°±0.2°, and 37.8°±0.2°;
the crystal form K has an X-ray powder diffraction (XRPD) pattern comprising characteristic peaks at 2θ angles of 7.6°±0.2°, 11.1°±0.2°, 12.1°±0.2°, and 14.3°±0.2°;
the crystal form L has an X-ray powder diffraction (XRPD) pattern comprising characteristic peaks at 2θ angles of 9.5°±0.2°, 12.3°±0.2°, 13.6°±0.2°, 14.5°±0.2°, 15.4°±0.2°, and 16.2°±0.2°.

2. The crystal form according to claim 1, wherein,
the X-ray powder diffraction (XRPD) pattern of the crystal form A further comprises characteristic peaks at 2θ angles of 3.6°±0.2°, 12.8°±0.2°, 13.2°±0.2°, 13.6°±0.2°, 15.2°±0.2°, 16.1°±0.2°, 18.3°±0.2°, and 24.1°±0.2°;
the X-ray powder diffraction (XRPD) pattern of the crystal form B further comprises characteristic peaks at 2θ angles of 4.8°±0.2°, 8.1°±0.2°, and 14.6°±0.2°;
the X-ray powder diffraction (XRPD) pattern of the crystal form C further comprises characteristic peaks at 2θ angles of 3.9°±0.2°, 8.3°±0.2°, 13.2°±0.2°, 19.5°±0.2°, and 20.3°±0.2°;
the X-ray powder diffraction (XRPD) pattern of the crystal form D further comprises characteristic peaks at 2θ angles of 4.5°±0.2°, 7.2°±0.2°, 12.9°±0.2°, and 19.0°±0.2°;
the X-ray powder diffraction (XRPD) pattern of the crystal form E further comprises characteristic peaks at 2θ angles of 4.5°±0.2°, 7.7°±0.2°, 13.6°±0.2°, 18.1°±0.2°, 19.8°±0.2°, 20.8°±0.2°, and 28.0°±0.2°;
the X-ray powder diffraction (XRPD) pattern of the crystal form G further comprises characteristic peaks at 2θ angles of 3.4°±0.2°, 7.3°±0.2°, 12.0°±0.2°, 14.4°±0.2°, 17.0°±0.2°, 18.8°±0.2°, 22.5°±0.2°, 25.5°±0.2°, 25.7°±0.2°, and 26.1°±0.2°;
the X-ray powder diffraction (XRPD) pattern of the crystal form J further comprises characteristic peaks at 2θ angles of 3.5°±0.2°, 4.7°±0.2°, 12.7°±0.2°, 13.7°±0.2°, 15.5°±0.2°, 16.2°±0.2°, and 18.1°±0.2°;
the X-ray powder diffraction (XRPD) pattern of the crystal form K further comprises characteristic peaks at 2θ angles of 4.5°±0.2°, 4.9°±0.2°, 5.5°±0.2°, 8.2°±0.2°, 13.5°±0.2°, and 18.7°±0.2°;
the X-ray powder diffraction (XRPD) pattern of the crystal form L further comprises characteristic peaks at 2θ angles of 3.7±0.2°, 7.9°±0.2°, 16.9°±0.2°, 17.3°±0.2°, 18.8°±0.2°, and 20.4°±0.2°.

3. The crystal form according to claim 1 or 2, wherein:
the X-ray powder diffraction (XRPD) pattern of the crystal form A comprises characteristic peaks at 2θ angles shown in Table 1;
the X-ray powder diffraction (XRPD) pattern of the crystal form B comprises characteristic peaks at 2θ angles shown in Table 14;
the X-ray powder diffraction (XRPD) pattern of the crystal form C comprises characteristic peaks at 2θ angles shown in Table 2;
the X-ray powder diffraction (XRPD) pattern of the crystal form D comprises characteristic peaks at 2θ angles shown in Table 15;
the X-ray powder diffraction (XRPD) pattern of the crystal form E comprises characteristic peaks at 2θ angles shown in Table 16;
the X-ray powder diffraction (XRPD) pattern of the crystal form G comprises characteristic peaks at 2θ angles shown in Table 17;
the X-ray powder diffraction (XRPD) pattern of the crystal form J comprises characteristic peaks at 2θ angles shown in Table 3;
the X-ray powder diffraction (XRPD) pattern of the crystal form K comprises characteristic peaks at 2θ angles shown in Table 20;
the X-ray powder diffraction (XRPD) pattern of the crystal form L comprises characteristic peaks at 2θ angles shown in Table 21.

4. The crystal form according to claim 1, wherein the crystal form A has any one or more of the following characteristics:
(1) having an X-ray powder diffraction (XRPD) pattern substantially as shown in FIG. 1a;
(2) having a differential scanning calorimetry (DSC) graph substantially as shown in FIG. 1b;
(3) having a thermogravimetric analysis (TGA) graph substantially as shown in FIG. 1c; and
(4) having a dynamic vapor sorption (DVS) graph substantially as shown in FIG. 1d.

5. The crystal form according to claim 1, wherein the crystal form C has any one or more of the following characteristics:
(1) having an X-ray powder diffraction (XRPD) pattern substantially as shown in FIG. 2a;
(2) having a differential scanning calorimetry (DSC) graph substantially as shown in FIG. 2b;
(3) having a thermogravimetric analysis (TGA) graph substantially as shown in FIG. 2c; and
(4) having a dynamic vapor sorption (DVS) graph substantially as shown in FIG. 2d.

6. The crystal form according to claim 1, wherein the crystal form J has any one or more of the following characteristics:
(1) having an X-ray powder diffraction (XRPD) pattern substantially as shown in FIG. 3a;
(2) having a differential scanning calorimetry (DSC) graph substantially as shown in FIG. 3b;
(3) having a thermogravimetric analysis (TGA) graph substantially as shown in FIG. 3c; and
(4) having a dynamic vapor sorption (DVS) graph substantially as shown in FIG. 3d.

7. A method for preparing a crystal form A of a Kudinoside A compound, wherein the method comprises:
subjecting *kudingcha* to percolation extraction and concentrating to obtain an extract in the form of an extractum; diluting and centrifuging the extractum to obtain a crude sample; purifying the crude sample by high-pressure chromatography, decoloring, recrystallizing, freeze-drying, and finally crushing to obtain the crystal form A; or
the crystal form A is prepared by any one of the following three methods:
method 1: dissolving amorphous Kudinoside A in an alcohol solvent to obtain a dissolution solution of Kudinoside A; concentrating the dissolution solution until a white precipitate is separated out and no liquid drops are generated at an inlet of a distilled liquid collecting container, thus reaching the end point of concentration and obtaining a concentrated solution; and freeze-drying the concentrated solution to obtain the crystal form A;
method 2: dissolving amorphous Kudinoside A in an alcohol solvent to obtain a dissolution solution of Kudinoside A; subjecting the dissolution solution to sonication treatment and filtering to obtain a filtrate; and concentrating the filtrate under reduced pressure to obtain a dry solid, namely the crystal form A;
method 3: dissolving amorphous Kudinoside A in an alcohol solvent to obtain a dissolution solution of Kudinoside A, and naturally volatilizing the dissolution solution at room temperature to obtain a dry solid, namely the crystal form A.

8. The method according to claim 7, wherein the percolation extraction is carried out using pure ethanol or 50-90% (v/v) aqueous ethanol solution, and the mass ratio of *kudingcha* powder to the alcohol is 1:2.5-1:1.5; the extract in the form of the extractum is diluted with water and anhydrous ethanol successively and centrifuged to obtain the crude sample, wherein the amount of water is 0.5-1 times the mass of the *kudingcha* powder, and the amount of the anhydrous ethanol is 0.3-0.8 times the mass of the *kudingcha* powder; the crude sample is dissolved in ethanol or an aqueous solution thereof at 60-90 °C and recrystallized at a temperature not more than 10 °C; the resulting crystal is freeze-dried at about 0 °C, then the temperature is raised to room temperature for drying the crystal, and finally the crystal is dried at a temperature not less than 32 °C to obtain the crystal form A, wherein the freeze-drying lasts for 1-3 h, the room temperature drying lasts for 3-5 h, and the drying at a temperature not less than 32 °C lasts for 5-8 h.

9. The method according to claim 7, wherein an aqueous ethanol solution is used to dissolve the amorphous Kudinoside A in the methods 1 and 2; in the method 3, methanol is used to dissolve the amorphous Kudinoside A; in the method 1, after the dissolution solution of the Kudinoside A is obtained, firstly filtering is carried out to obtain a clear solution, and then the clear solution is concentrated, wherein the concentrating is carried out in a water bath at 50-60 °C, the rotating speed is controlled to be 30-80 rpm, and the vacuum degree is controlled to be not lower than 0.08 MPa.

10. A method for preparing a crystal form C or J of a Kudinoside A compound, wherein,
the crystal form C is prepared by any one of the following methods 4-6:
method 4: dissolving a crystal form A in a solvent to obtain a suspension; stirring for 3-5 days at room temperature and centrifuging; and drying the resulting solid under vacuum at 25-30 °C overnight to obtain the crystal form C;
method 5: dissolving a crystal form A in ethyl acetate to obtain a suspension; stirring for 2-4 days at room temperature and centrifuging; and drying the resulting solid under vacuum at 25-30 °C overnight to obtain the crystal form C;
method 6: heating a crystal form A for 3-10 min at 150-200 °C to obtain the crystal form C;
the crystal form J is prepared by any one of the following methods 7-10:
method 7: dissolving a crystal form A in a saturated aqueous ethyl acetate solution to obtain a suspension; stirring for 3-5 days at room temperature and centrifuging; and drying the resulting solid under vacuum at 25-30 °C overnight to obtain the crystal form J;
method 8: dissolving a crystal form A in water to obtain a suspension; stirring for 2-4 days at room temperature and centrifuging; and drying the resulting solid under vacuum at 25-30 °C overnight to obtain the crystal form J;
method 9: dissolving amorphous Kudinoside A in an alcohol solvent; adding water, stirring overnight at room temperature, and centrifuging; and drying the resulting solid under vacuum at 25-30 °C overnight to obtain the crystal form J;
method 10: dissolving amorphous Kudinoside A in an alcohol solvent and water; stirring overnight at 0-10 °C and centrifuging; and drying the resulting solid under vacuum at 25-30 °C overnight to obtain the crystal form J.

11. The method according to claim 10, wherein,
in the method 4, the volume ratio of acetonitrile to isopropyl ether is 1:1 to 1:5; the mass-to-volume ratio (g:mL) of the crystal form A to the solvent is 1:20 to 1: 50;
in the method 5, the mass-to-volume ratio (g:mL) of the crystal form A to ethyl acetate is 1:10 to 1:30;
in the method 6, the crystal form A is heated at 170±5 °C and subjected to heat preservation for 3-7 min;
in the method 7, the mass-to-volume ratio (g:mL) of the crystal form A to the saturated aqueous ethyl acetate solution is 1:20 to 1:50;
in the method 8, the mass-to-volume ratio (g:mL) of the crystal form A to water is 1:10 to 1:30;
in the method 9, the alcohol solvent is an anhydrous alcohol solvent, the mass-to-volume ratio (g:mL) of the Kudinoside A to the alcohol solvent is 1:30 to 1:100, and the amount of water added is 1-10 times the volume of the alcohol solvent;
in the method 10, the alcohol solvent is preferably an anhydrous alcohol solvent, the volume ratio of the alcohol solvent to water is 1:1 to 1:5, and the mass-to-volume ratio (g:mL) of the Kudinoside A to the alcohol solvent is 1:10 to 1:50.

12. Use of the crystal form of the Kudinoside A compound according to any one of claims 1-6 or a product prepared by the method according to any one of claims 7-11 in preparing a medicament for dilating trachea, resisting inflammation, or treating a pulmonary disease, wherein,
preferably, the pulmonary disease is chronic obstructive pulmonary disease or asthma;
preferably, the crystal form is a crystal form A, a crystal form C, or a crystal form J, or any mixture thereof;
preferably, the medicament is an inhalation formulation; preferably, the inhalation formulation is an inhalation powder, an inhalation aerosol, an inhalation spray, an inhalation suspension, or an inhalation solution.

13. A pharmaceutical composition for treating a pulmonary disease, comprising a therapeutically effective amount of the crystal form of the Kudinoside A compound according to any one of claims 1-6 or a product prepared by the method according to any one of claims 7-11 and a pharmaceutically acceptable carrier, wherein,
preferably, the crystal form is a crystal form A, a crystal form C, or a crystal form J, or any mixture thereof, and the product comprises the crystal form A, the crystal form C, or the crystal form J, or any mixture thereof;
preferably, the pharmaceutical composition is an inhalation formulation; preferably, the inhalation formulation is an inhalation powder, an inhalation aerosol, an inhalation spray, an inhalation suspension, or an inhalation solution.

14. A method for dilating trachea, resisting inflammation, or treating a pulmonary disease, comprising administering to a patient in need thereof an effective amount of the crystal form of the Kudinoside A compound according to any one of claims 1-6, a product prepared by the method according to any one of claims 7-11, or a pharmaceutical composition comprising the crystal form or the product, wherein preferably, the crystal form is a crystal form A, a crystal form C, or a crystal form J, or any mixture thereof, and the product comprises the crystal form A, the crystal form C, or the crystal form J, or any mixture thereof.

15. The method according to claim 14, wherein the pulmonary disease is chronic obstructive pulmonary disease or asthma.
